# EUROPÄISCHE PATENTSCHRIFT

(11) **EP 3 728 371 B1**
(45) Veröffentlichungstag und Bekanntmachung des Hinweises auf die Patenterteilung: **12.01.2022**
(21) Anmeldenummer: 18819100.1
(22) Anmeldetag: 13.12.2018
(51) Int. Cl.: C08G 18/48, C08G 18/66, C08G 18/73, C08G 18/75, C08G 18/08, C08G 18/24, C08G 18/32, C08G 18/38, C09J 175/02, C09J 175/08

(54) **KLEBSTOFF AUF BASIS EINES SPEZIELLEN POLYURETHANHARNSTOFFS MIT EINSTELLBARER KLEBKRAFT SOWIE DESSEN HERSTELLUNG UND ANWENDUNG**
ADHESIVE BASED ON A SPECIAL POLYURETHANE UREA WITH ADJUSTABLE ADHESIVE FORCE AND USE AND MANUFACTURE OF SAME
ADHÉSIF À BASE D'UNE URÉE DE POLYURETHANE SPÉCIALE À FORCE D'ADHÉSION RÉGLABLE AINSI QUE SA FABRICATION ET SON APPLICATION

(30) Priorität: 21.12.2017 EP 17209627
(43) Veröffentlichungstag der Anmeldung: 28.10.2020
(73) Patentinhaber: Covestro Deutschland AG, 51373 Leverkusen (DE)
(72) Erfinder: WEISER, Marc-Stephan, 51515 Kürten-Dürscheid (DE); PLUG, Sascha, 51375 Leverkusen (DE); DÖRR, Sebastian, 40593 Düsseldorf (DE)
(74) Vertreter: Levpat
(86) Internationale Anmeldenummer: PCT/EP2018/084672
(87) Internationale Veröffentlichungsnummer: WO 2019/121282

(56) Entgegenhaltungen:
- DE-A1-102007 052 966
- US-B1- 6 642 304

## Beschreibung

Die vorliegende Erfindung betrifft einen bevorzugt amorphen Klebstoff, herstellbar aus einer wässrigen Polyurethanharnstoff-Dispersion, enthaltend einen speziellen bevorzugt amorphen Polyurethanharnstoff mit einstellbarer Klebkraft, sowie eine Klebstoffschicht und ein Produkt enthaltend den Klebstoff. Ebenfalls Gegenstand der Erfindung ist eine wässrige Dispersion enthaltend den speziellen, bevorzugt amorphen Polyurethanharnstoff sowie dessen möglichen Verwendungen. Dieser, bevorzugt amorphe Klebstoff lässt sich auf sehr effiziente Weise herstellen.

In vielen, insbesondere medizinischen Anwendungen, wie beispielsweise selbstklebenden Bandagen, Pflastern oder anderen Wundabdeckungsmitteln kommen selbstklebende Klebstoffe zum Einsatz. Die Anforderungen an selbstklebende Klebstoffe können sehr vielfältig sein. Den selbstklebenden Klebstoffen ist gemein, dass der Klebstoff zwar gut auf der zu fixierenden Oberfläche haftet, aber gleichzeitig gut wieder entfernt werden kann, möglichst ohne Rückstände. In der medizinischen Anwendung ist es sehr vorteilhaft, einen Klebstoff zur Fixierung unterschiedlichster Gegenstände, wie Pflaster, Bandagen, Tapes oder andere Wundabdeckungsmittel bereitzustellen, die einerseits gut über einen langen Zeitraum, wie beispielsweise mehrere Tage oder Wochen haften, aber nach der Tragezeit ohne Schädigung der oberen Hautschichten und bevorzugt ohne Schmerzen wieder entfernbar sind. Darüber hinaus sollte der Klebstoff keine Rückstände nach dessen Entfernung auf der Haut zurücklassen, keine Allergien auslösen, um Hautreizungen zu vermeiden, atmungsaktiv sein, dabei wasserstabil und eine gute Haftung auf dem Trägermaterial des Klebers (z.B. Folie) haben.

Oft werden in solchen Produkten selbstklebende Acrylat- oder Silikonklebstoffe verwendet. Während Acrylatklebstoffe auch hohe Klebstärken ermöglichen, sind sie häufig durch ihr thermoplastisches Fließverhalten auf der Haut nach längerer Tragezeit nur unter Schädigung der obersten Hautschichten und mit großen Schmerz wieder abziehbar, sie führen zu starken Hautirritationen und ggf. zu allergenen Reaktionen. Silikonklebstoffe dagegen sind häufig nur von geringerer Klebkraft und ermöglichen daher für verschiedene medizinische Anwendungen wie NPWT (Unterdruck-Wundtherapie oder Vakuumtherapie), Ostomie (künstlicher Darmausgang) und verschiedene medizinische Klebebänder, auch Tapes genannt, z.B. chirurgische Tapes, keine ausreichend hohe Klebkraft für eine sichere Verklebung, insbesondere über einen längeren Zeitraum.

Generell muss dabei eine gute Balance aus ausreichender Klebkraft für eine ausreichend lange Tragezeit und sanftem Ablösen des Klebstoffs beim Entfernen des Verbands von der Haut gefunden werden. Dies ist von Anwendung zu Anwendung verschieden. Klebstoffe auf Basis der unveröffentlichten europäischen Patentanmeldung mit der Anmeldenummer EP16177199.3 ermöglichen jedoch keine einstellbare Klebkraft. Für die Verklebung auf Wunden, sensibler oder brüchiger Haut sind die Klebstoffe aus der EP16177199.3 durch ihre hohe Klebstärke und dem Aufbau der Klebkraft über die Zeit wenig geeignet. Dagegen sind bisherige Klebstoffe im Markt wie die in EP897406, EP147588 und EP57839 beschriebenen Systeme nur durch einen komplizierten Zweikomponenten-Mischprozess herzustellen, was für typische Klebstoffhersteller eine komplexe und oft schwer zu beherrschende Vorgehensweise ist.

Eine Aufgabe der vorliegenden Erfindung ist es, wenigstens einen Teil der Nachteile des Standes der Technik wenigstens zu einem Teil zu verbessern.

Weiterhin ist eine Aufgabe der vorliegenden Erfindung einen hautfreundlichen Haftklebstoff für medizinische Anwendung mit einer ausreichend hohen Klebkraft bereitzustellen, insbesondere in einem Bereich von 0,3 N/20 mm bis 25 N/20 mm zu definieren gegen Aluminium gemäß DIN EN 1464 (90° Rollenschälprüfung) auf einer Zugprüfmaschine gemäß DIN EN ISO 527-1.

Eine weitere Aufgabe der Erfindung ist es, einen Klebstoff, beispielsweise in Form einer Klebstoffschicht bereitzustellen, die eine einfache und kostengünstige Herstellweise erlaubt.

Es ist weiterhin eine Aufgabe der Erfindung einen Klebstoff bereitzustellen, der eine möglichst einfache Verarbeitbarkeit erlaubt, also über eine möglichst lange "Potlife" verfügt, so dass direkt am Herstellungsort bevorzugt aus einem einzigen Gefäß bevorzugt für mehrere Stunden mit der vollständigen Mischung aller Komponenten der Formulierung heraus gearbeitet werden kann.

Weiterhin ist eine Aufgabe der Erfindung einen Klebstoff, beispielsweise in Form einer Klebstoffschicht bereitzustellen, der eine gute Hautverträglichkeit, bei hohem Tragekomfort und guter Wiederentfernbarkeit aufweist. Insbesondere sollte der Tragekomfort und die rückstandsfreie Wiederentfernbarkeit auch nach mehreren Wochen Tragezeit, insbesondere auf Wunden und auf brüchiger oder sensibler Haut noch gewährleistet sein.

Wiederum eine Aufgabe der Erfindung ist es, einen Klebstoff beispielsweise in Form einer Klebstoffschicht bereitzustellen, der eine hohe Klebkraft kombiniert mit einer guten Hautverträglichkeit und möglichst guter Wiederentfernbarkeit, insbesondere auf Wunden und auf brüchiger oder sensibler Haut aufweist.

Eine weitere Aufgabe der Erfindung ist es, einen Klebstoff, beispielsweise in Form einer Klebstoffschicht, bereitzustellen, die die gleichen Vorteile, wie für den Klebstoff beschrieben aufweist und dabei in verschiedenen medizinischen aber auch technischen Anwendungen, beispielsweise sowohl im "professional woundcare" Bereich als auch im OTC woundcare Bereich einsetzbar ist.

Weiterhin ist eine Aufgabe der Erfindung eine wässrige Polyurethanharnstoff-Dispersion bereitzustellen, aus der auf einfache Weise der Klebstoff bzw. die Klebstoffschicht gemäß der Erfindung gewonnen werden kann.

Es ist weiterhin eine Aufgabe der Erfindung, ein Verfahren zu Herstellung einer Klebstoffschicht bereitzustellen, das die erfindungsgemäße Polyurethanharnstoff-Dispersion enthält und sämtliche Vorteile der erfindungsgemäßen Klebstoffschicht aufweist.

Eine Aufgabe der Erfindung ist es, eine Verwendung des Klebstoffs bzw. der Klebstoffschicht zur Befestigung von Gegenständen vorzugsweise auf der Haut bereitzustellen, wobei der Klebstoff bzw. die Klebstoffschicht die bereits erwähnten Vorteile in den Gegenstand bzw. der Befestigung einbringt.

Eine weitere Aufgabe der Erfindung ist es, eine Verwendung einer Polyurethanharnstoff-Dispersion zur Herstellung eines Klebstoffs, einer Klebstoffschicht oder eines Produktes bereitzustellen, die die bereits erwähnten Vorteile aufweisen.

Mindestens eine der Aufgaben wird gelöst durch einen Klebstoff gemäß des Gegenstands des Anspruch 1. Besondere Ausführungsformen sind in den abhängigen Ansprüchen beschrieben. Weiterhin ist mindestens ein Teil der Aufgaben durch eine Klebstoffschicht oder ein Produkt beinhaltend den erfindungsgemäßen Klebstoff gelöst. Wiederum ein Teil der Aufgaben wird durch die Ausführung des Verfahrens zur Herstellung der Klebstoffschicht gelöst.

Ein erster Gegenstand der Erfindung betrifft einen Klebstoff herstellbar aus einer wässrigen Polyurethanharnstoff-Dispersion, enthaltend
(V1) einen bevorzugt amorphen Polyurethanharnstoff (V1), welcher erhältlich ist, durch Umsetzung von wenigstens
   A) einer aliphatischen, cycloaliphatischen oder araliphatischen Polyisocyanat-Komponente mit einer mittleren Isocyanatfunktionalität von ≥ 1,8 und ≤ 2,6,
   B) einer polymeren Polyether-Polyol-Komponente,
   C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
   D) gegebenenfalls weiteren hydrophilierenden Komponenten, die unterschiedlich sind von C2),
   E) gegebenenfalls hydroxyfunktionellen Verbindungen mit einem Molekulargewicht von 62 bis 399 mol/g,
   F) gegebenenfalls weitere polymere Polyole, welche unterschiedlich sind von B),
   G) gegebenenfalls einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
   H) gegebenenfalls einer aliphatischen, cycloaliphatischen oder araliphatischen Polyisocyanat-Komponente mit einer mittleren Isocyanatfunktionalität von > 2,6 und ≤ 4,
   wobei die Komponenten B) und F) zusammen ≤ 30 Gew.-% an Komponente F), bezogen auf die Gesamtmasse der Komponenten B) und F) enthalten, sowie
(V2) ein hydrophiles Polyisocyanat, herstellbar mindestens aus den Komponenten
   I) eine aliphatische, cycloaliphatische oder araliphatische Polyisocyanat-Komponente mit einer mittleren Isocyanatfunktionalität von bevorzugt ≥ 2,0 und ≤ 3,6
   J) eine polymere, hydrophile und monofunktionelle Polyalkylenoxid-Komponente,
   K) gegebenenfalls weiteren, bevorzugt hydrophilierenden Komponenten, die unterschiedlich sind von J),
   L) gegebenenfalls Zuschlags- und Hilfsstoffen.

Bevorzugt weist der Klebstoff ein Verhältnis des Polyurethanharnstoff (V1) zu dem Polyisocyanat (V2) in einem Bereich von 80:1 bis 1,2:1 oder bevorzugt in einem Bereich von 60:1 bis 2:1, oder bevorzugt in einem Bereich von 40:1 bis 4:1 auf.

Bevorzugt ist der Klebstoff amorph. Amorph bedeutet im Sinne dieser Erfindung, dass der Polyurethanharnstoff im in der im folgenden ausgeführten Messmethode genannten Temperaturbereich keine oder nur so geringe kristalline Anteile ausbildet, dass mittels der beschriebenen DSC Messungen nur ein oder mehrere Glasübergangspunkte T_{g}, aber keine Schmelzbereiche mit einer Schmelzenthalpie ≥ 20 J/g in dem genannten Temperaturbereich gefunden werden können

Bevorzugt weist die wässrige Polyurethanharnstoff-Dispersion ein Verhältnis des Polyurethanharnstoff (V1) zu dem Polyisocyanat (V2) in einem Bereich von 200:1 bis 3:1 oder bevorzugt in einem Bereich von 150:1 bis 15:1, oder bevorzugt in einem Bereich von 100:1 bis 10:1.

Bevorzugt amorphe Polyurethanharnstoffe im Sinne der Erfindung sind polymere Verbindungen, die mindestens zwei, bevorzugt mindestens drei Urethan-Gruppen-haltige Wiederholungseinheiten aufweisen:

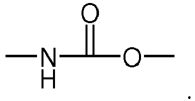

Erfindungsgemäß weisen die bevorzugt amorphen Polyurethanharnstoffe herstellungsbedingt auch Harnstoff-Gruppen-haltige Wiederholungseinheiten auf, 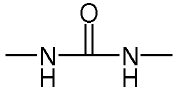 wie sie insbesondere bei der Umsetzung von isocyanatterminierten Präpolymeren mit aminofunktionellen Verbindungen gebildet werden.

Unter ionogenen Gruppen werden im Sinne dieser Erfindung solche funktionellen Gruppen verstanden, die in der Lage sind, beispielsweise durch Neutralisation mit einer Base, ionische Gruppen zu bilden.

Die Komponente A) kann jedes Polyisocyanat sein, das der Fachmann hierfür verwenden würde. Als Komponente A) bevorzugt geeignete Polyisocyanate sind insbesondere die dem Fachmann an sich bekannten aliphatischen Polyisocyanate mit einer mittleren Isocyanatfunktionalität von ≥ 1,8 und ≤ 2,6. Der Begriff aliphatisch umfasst dabei auch cycloaliphatische und/oder araliphatische Polyisocyanate.

Unter der mittleren Isocyanatfunktionalität wird dabei die durchschnittliche Anzahl an Isocyanatgruppen pro Molekül verstanden.

Bevorzugte Polyisocyanate sind solche des Molekulargewichtsbereichs von 140 bis 336 g/mol. Besonders bevorzugt sind diese ausgewählt aus der Gruppe bestehend aus. 1,4-Diisocyanatobutan (BDI), 1,5-Pentandiisocyanat, (PDI) 1,6-Diisocyanatohexan (HDI), 1,3-Bis(isocyanatomethyl)benzol (1,3-Xylylendiisocyanat, XDI), 1,4-Bis(isocyanatomethyl)benzol (1,4-Xylylendiisocyanat, XDI), 1,3-Bis(1-isocyanato-1 -methyl-ethyl)benzol (TMXDI), 1,4-Bis(1-isocyanato-1-methyl-ethyl)benzol (TMXDI), 4-Isocyanatomethyl-1,8-octandiisocyanat (Trisisocyanatononan (TIN)), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan sowie die cycloaliphatischen Diisocyanate 1,3- bzw. 1,4-Diisocyanatocyclohexan, 1,4-Diisocyanato-3,3,5-trimethylcyclohexan, 1,3-Diisocyanato-2(4)-methylcyclohexan, 1-Isocyanato-3,3,5-trimethyl-5-isocyanatomethylcyclohexan (Isophorondiisocyanat, IPDI), 1-Isocyanato-1-methyl-4(3)isocyanato-methylcyclohexan, 1,8-Diisocyanato-p-menthan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetramethyl-1,1'-bi(cyclohexyl), 4,4'- und/oder 2,4'-Diisocyanato-dicyclohexylmethan, 4,4'-Diisocyanato-3,3'-dimethyldicyclohexylmethan, 4,4'-Diisocyanato-3,3',5,5'-tetramethyldicyclohexylmethan, 1,3-Diisocyanatoadamantan, und 1,3-Dimethyl-5,7-diisocyanatoadamantan oder beliebige Gemische solcher Isocyanate. Ganz besonders bevorzugt sind die Polyisocyanate ausgewählt aus 1,4-Butylendiisocyanat, 1,5-Pentamethylendiisocyanat (PDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts (H12-MDI), 1,4-Cyclohexylendiisocyanat, 4-Isocyanatomethyl-1,8-octandiisocyanat (Nonantriisocyanat) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate, die eine mittlere Isocyanatfunktionalität ≥ 2 und ≤ 2,6 aufweisen, mit Uretdion-, Isocyanurat-, Urethan-, Allophanat-, Biuret-, Iminooxadiazindion- oder Oxadiazintrionstruktur sowie Mischungen aus diesen und/oder den oben stehenden anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von ≥ 1,8 und ≤ 2,6 und besonders bevorzugt ≥ 2,0 und ≤ 2,4.

In einer bevorzugten Ausführungsform des Klebstoffes enthält die Komponente A) ein aliphatisches oder cycloaliphatisches Polyisocyanat ausgewählt aus der Gruppe bestehend aus HDI, IPDI und/oder H12-MDI oder deren Modifikationsprodukten, ganz besonders bevorzugt ausgewählt aus HDI und/oder IPDI.

In einer insbesondere bevorzugten Variante liegen als Komponente A) IPDI und HDI im Gemisch vor.

Bevorzugt liegt das Gewichtsverhältnis von IPDI:HDI für die Polyisocyanat-Komponente A) in einem Bereich von 1,05 bis 10, besonders bevorzugt in einem Bereich von 1,1 bis 5, und ganz besonders bevorzugt in einem Bereich von 1,1 bis 1,5.

In einer bevorzugten Ausführungsform wird zur Herstellung des erfindungsgemäß verwendeten bevorzugt amorphen Polyurethanharnstoffs ≥ 5 und ≤ 40 Gew.-% der Komponente A) und besonders bevorzugt ≥ 10 und ≤ 35 Gew.-% der Komponente A), jeweils bezogen auf die Gesamtmasse des bevorzugt amorphen Polyurethanharnstoffs, eingesetzt.

In einer weiteren bevorzugten Ausführungsform wird zur Herstellung des bevorzugt amorphen Polyurethanharnstoffs auch die Komponente H), eine aliphatische, cycloaliphatische oder araliphatische Polyisocyanat-Komponente mit einer mittleren Isocyanatfunktionalität (mittlere Anzahl Isocyanatgruppen pro Molekül) von > 2,6 und ≤ 4, vorzugsweise ≥ 2,8 und ≤ 3,8 eingesetzt. Die Komponente H) wird dabei bevorzugt im Gemisch mit Komponente A) eingesetzt.

Als Komponente H) besonders geeignet sind oligomere Diisocyanate, die eine Funktionalität > 2,6 und ≤ 4, vorzugsweise ≥ 2,8 und ≤ 3,8 aufweisen, mit Isocyanurat-, Urethan-, Allophanat-, Biuret-, Iminooxadiazindion- oder Oxadiazintrionstruktur. Ganz besonders bevorzugt enthält H) Isocyanuratstrukturen.

Bevorzugt besteht die aliphatische, cycloaliphatische oder araliphatische Polyisocyanat-Komponente H) aus einem aliphatischen oder cycloaliphatischen Polyisocyanat-Oligomer basierend auf HDI, IPDI und/oder H12-MDI, ganz besonders bevorzugt basierend auf HDI.

Das Molverhältnis der NCO-Gruppen aus Komponente A) zu Komponente H) beträgt dabei bevorzugt 100:0,5 bis 100: 50; besonders bevorzugt 100: 2 bis 100: 15 und ganz besonders bevorzugt 100:3 bis 100: 8.

In einer bevorzugten Ausführungsform wird zur Herstellung des erfindungsgemäß verwendeten bevorzugt amorphen Polyurethanharnstoffs ≥ 0 und ≤ 10 Gew.-% der Komponente H) und besonders bevorzugt ≥ 0,1 und ≤ 3 Gew.-% der Komponente H), jeweils bezogen auf die Gesamtmasse des bevorzugt amorphen Polyurethanharnstoffs, eingesetzt.

Die erfindungsgemäß als Komponente B) eingesetzten polymeren Polyether-Polyole weisen bevorzugt zahlenmittlere Molekulargewichte in einem Bereich von 400 bis 8000 g/mol, bevorzugt in einem Bereich von 600 bis 6000 g/mol, oder bevorzugt in einem Bereich von 1000 bis 3000 g/mol, bestimmt durch Gelpermeationschromatographie gegenüber Polystyrol-Standard in Tetrahydrofuran bei 23°C, und/oder einer OH-Funktionalität von bevorzugt in einem Bereich von 1,5 bis 6, bevorzugter in einem Bereich von 1,8 bis 3, besonders bevorzugt von in einem Bereich von 1,9 bis 2,1 auf. Der Ausdruck "polymere" Polyether-Polyole bedeutet hier insbesondere, dass die genannten Polyole mindestens zwei, bevorzugt mindestens drei miteinander verbundene Wiederholungseinheiten aufweisen.

Das zahlenmittlere Molekulargewicht wird im Rahmen dieser Anmeldung stets bestimmt durch Gelpermeationschromatographie (GPC) in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2xPSS SDV linear M, 8x300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Geeignete Polyetherpolyole sind beispielsweise die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten. Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

In einer bevorzugten Ausführungsform des Klebstoffes enthält die Komponente B) Poly(propylenglykol)polyetherpolyole. Bevorzugt beinhaltet der Klebstoff Poly(propylenglykol)-polyetherpolyole in einem Bereich von 50 bis 100 Gew.-%, oder bevorzugt in einem Bereich von 70 bis 100 Gew.-% oder bevorzugt in einem Bereich von 90 bis 100 Gew.-%, besonders bevorzugt zu 100 Gew.-%, jeweils bezogen auf das Gesamtgewicht der Komponente B).

Weist die Komponente B) mehr als 50 Gew.-% Poly(propylenglykol)polyetherpolyole auf, so wird nur gegebenenfalls eine geringe Menge an Komponente G) eingesetzt oder bevorzugt Komponente G) gar nicht eingesetzt. Sollte die Komponente B) zur Herstellung des Klebstoffes mehr als 10 Gew.-%, bevorzugt mehr als 20 Gew.-%, oder bevorzugt mehr als 30 Gew.-% Poly(tetramethylen)polyetherpolyole aufweisen, so ist es bevorzugt, dass Komponente G) anwesend ist.

In einer bevorzugten Ausführungsform des Klebstoffes weist die Komponente B) eine mittlere Molekularmasse in einem Bereich von 400 bis 4000 g/mol, oder bevorzugt in einem Bereich von 500 bis 3500 g/mol, oder bevorzugt in einem Bereich von 800 bis 3000 g/mol auf.

In einer weiteren bevorzugten Ausführungsform des Klebstoffes enthält die Komponente B) ein Gemisch aus Poly(propylenglykol)polyetherpolyole mit unterschiedlichem mittleren Molekulargewicht oder besteht daraus, wobei sich die Poly(propylenglykol)polyetherpolyole in ihren zahlenmittleren Molekulargewichten um wenigstens 100 g/mol, bevorzugt um wenigstens 200 g/mol, oder bevorzugt um wenigstens 400 g/mol, oder bevorzugt um wenigstens 800 g/mol, oder bevorzugt um wenigstens 1000 g/mol unterscheiden. Bevorzugt unterscheiden sich die zahlenmittleren Molekulargewichte der Poly(propylenglykol)polyetherpolyole um nicht mehr als 5000 g/mol, oder um nicht mehr als 4000 g/mol, oder um nicht mehr als 3000 g/mol.

In einer besonders bevorzugten Ausführungsform enthält die Komponente B) ein Gemisch aus Poly(propylenglykol)polyetherpolyolen I mit einem zahlenmittleren Molekulargewichte Mₙ von ≥ 400 und ≤ 1500 g/mol, besonders bevorzugt von ≥ 600 und ≤ 1200 g/mol, ganz besonders bevorzugt von 1000 g/mol und Poly(propylenglykol)polyetherpolyolen II mit einem zahlenmittleren Molekulargewichte Mₙ von ≥ 1500 und ≤ 8000 g/mol, besonders bevorzugt von ≥ 1800 und ≤ 3000 g/mol, ganz besonders bevorzugt von 2000 g/mol.

Das Gewichtsverhältnis der Poly(propylenglykol)polyetherpolyolen I zu den Poly(propylenglykol)polyetherpolyolen II liegt bevorzugt im Bereich von 0,01 bis 10, besonders bevorzugt im Bereich von 0,02 bis 5, ganz besonders bevorzugt im Bereich von 0,05 bis 1.

Erfindungsgemäß wird zur Herstellung des bevorzugt amorphen Polyurethanharnstoffs eine aminofunktionelle Kettenverlängerer-Komponente C) mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist, eingesetzt.

Die aminofunktionellen Verbindungen der Komponente C) Komponente werden bevorzugt aus primären und/oder sekundären Diaminen ausgewählt. Insbesondere umfassen die aminofunktionellen Verbindungen C) mindestens ein Diamin.

In einer bevorzugten Ausführungsform des Klebstoffes umfasst die aminofunktionelle Komponente C) wenigstens eine aminofunktionelle Verbindung C2), die ionische und/oder ionogene Gruppen aufweist.

In einer weiteren bevorzugten Ausführungsform der Erfindung umfasst die aminofunktionelle Komponente C) sowohl aminofunktionelle Verbindungen C2), die ionische und/oder ionogene Gruppe aufweisen, als auch aminofunktionelle Verbindungen C1), die keine ionische oder ionogene Gruppe aufweisen.

Beispielsweise können als Komponente C1) organische Di- oder Polyamine wie beispielsweise 1,2-Ethylendiamin, 1,2-und 1,3-Diaminopropan, 1,4-Diaminobutan, 1,6-Diaminohexan, Isophorondiamin (IPDA), Isomerengemisch von 2,2,4- und 2,4,4-Trimethylhexamethylendiamin, 2-Methylpentamethylendiamin, Diethylentriamin, 4,4-Diaminodicyclohexylmethan und/oder Dimethylethylendiamin oder Mischungen aus mindestens zwei hiervon eingesetzt werden.

Bevorzugt ist die Komponente C1) ausgewählt aus der Gruppe bestehend aus 1,2-Ethylendiamin, Bis(4-aminocyclohexyl)methan, 1,4-Diaminobutan, IPDA, Ethanolamin, Diethanolamin und Diethylentriamin oder einer Mischung aus mindestens zwei hiervon.

In einer weiteren bevorzugten Ausführungsform enthält die Komponente C1) > 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin oder IPDA oder ein Gemisch aus 1,2-Ethylendiamin und IPDA, wobei die Summe der beiden Amine in Bezug auf die Gesamtmenge an C1) bevorzugt in den genannten Anteilen vorliegt.

Bevorzugt umfasst die hydrophilierende Komponente C2) mindestens eine anionisch hydrophilierende Verbindung. Weiterhin bevorzugt beinhaltet die hydrophilierende Komponente C2) eine anionisch hydrophilierende Verbindung zu mindestens 80 Gew.-%, oder bevorzugt zu mindestens 90 Gew.-%, bezogen auf das Gesamtgewicht der Komponente C2). Besonders bevorzugt besteht die Komponente C2) aus ausschließlich anionisch hydrophilierenden Verbindungen.

Geeignete anionisch hydrophilierende Verbindungen enthalten wenigstens eine anionische oder ionogene Gruppe, die in eine anionische Gruppe überführt werden kann. Des Weiteren bevorzugt weisen geeignete anionisch hydrophilierende Verbindungen wenigstens zwei Aminogruppen und besonders bevorzugt zwei Aminogruppen auf. Besonders bevorzugt umfasst die hydrophilierende Komponente C2) eine anionisch hydrophilierende Verbindung, die wenigstens eine anionische oder ionogene Gruppe und wenigstens zwei Aminogruppen aufweist oder besteht daraus.

Geeignete anionisch hydrophilierende Verbindungen als Komponente C2), im Weiteren auch Hydrophilierungsmittel C2) genannt, enthalten bevorzugt eine Sulfonsäure- oder Sulfonatgruppe, besonders bevorzugt eine Natriumsulfonatgruppe. Geeignete anionisch hydrophilierende Verbindungen als Komponente C2) sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure oder 1,2- oder 1,3-Propylendiamin-β-ethylsulfonsäure oder Mischungen aus mindestens zwei hiervon.

Besonders bevorzugte anionische Hydrophilierungsmittel C2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure. Ganz besonders bevorzugt wird 2-(2-Aminoethylamino)ethylsulfonsäure oder deren Salze als anionisches Hydrophilierungsmittel C2) eingesetzt.

Gegebenenfalls kann die anionische Gruppe in der Komponente C2) auch eine Carboxylat- bzw. Carbonsäuregruppe sein. Die Komponente C2) wird dann bevorzugt aus Diaminocarbonsäuren ausgewählt. Bei dieser alternativen Ausführungsform müssen jedoch die Carbonsäure-basierenden Komponenten C2) in höheren Konzentrationen eingesetzt werden, verglichen mit solchen Komponenten C2), die Sulfonat- oder Sulfonsäuregruppen tragen. Besonders bevorzugt werden daher zur Herstellung des bevorzugt amorphen Polyurethanharnstoffs keine hydrophilierenden Verbindungen, die ausschließlich Carboxylatgruppen als anionische Gruppen der Komponente C2) tragen eingesetzt.

In einer bevorzugten Ausführungsform wird zur Herstellung des erfindungsgemäß verwendeten bevorzugt amorphen Polyurethanharnstoffs ≥ 0,1 und ≤ 10 Gew.-% der Komponente C2) und besonders bevorzugt ≥ 0,5 und ≤ 4 Gew.-% der Komponente C2), jeweils bezogen auf die Gesamtmasse des bevorzugt amorphen Polyurethanharnstoffs, eingesetzt.

Zur Hydrophilierung können auch Mischungen aus anionischen Hydrophilierungsmitteln C2) und weiteren Hydrophilierungsmitteln D), welche von C2) verschieden sind, verwendet werden.

Geeignete weitere Hydrophilierungsmittel D) sind beispielsweise nichtionische hydrophilierende Verbindungen D1) und/oder hydroxyfunktionelle ionische oder ionogene Hydrophilierungsmittel D2). In einer bevorzugten Ausführungsform des Klebstoffes handelt es sich bei der Komponente D) um nichtionisch hydrophilierende Komponenten D1).

Geeignete hydroxyfunktionelle ionische oder ionogene Hydrophilierungsmittel als Komponente D2) sind beispielsweise Hydroxycarbonsäuren wie Mono- und Dihydroxycarbonsäuren, wie 2-Hydroxyessigsäure, 3-Hydroxypropansäure, 12-Hydroxy-9-octadecansäure (Rizinolsäure), Hydroxypivalinsäure, Milchsäure, Dimethylolbuttersäure und/oder Dimethylolpropionsäure oder Gemische von mindestens zwei hieraus. Bevorzugt sind Hydroxypivalinsäure, Milchsäure und/oder Dimethylolpropionsäure, besonders bevorzugt ist Dimethylolpropionsäure. Bevorzugt werden keine hydroxyfunktionellen ionische oder ionogenen Hydrophilierungsmittel D2), insbesondere bevorzugt keine Hydrophilierungsmittel, die Carboxylat und Hydroxygruppen aufweisen, wie beispielsweise Dimethylolpropionsäure eingesetzt. Bevorzugt ist die Menge an hydroxyfunktionellen ionischen oder ionogenen Hydrophilierungsmittel D2) in einem Bereich von 0 bis 1 Gew.-%, oder bevorzugt in einem Bereich von 0,01 bis 0,5 Gew.-%, bezogen auf die Gesamtmasse des bevorzugt amorphen Polyurethanharnstoffs in dem bevorzugt amorphen Polyurethanharnstoff enthalten.

Geeignete nichtionisch hydrophilierende Verbindungen als Komponente D1) sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether, Methanol oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

In einer bevorzugten Ausführungsform der Erfindung enthält der erfindungsgemäß verwendete bevorzugt amorphen Polyurethanharnstoff ≥ 0 und ≤ 20 Gew.-% der Komponente D), bevorzugt ≥ 0,1 und ≤ 10 Gew.-% der Komponente D) und ganz besonders bevorzugt ≥ 1 und ≤ 5 Gew.-% der Komponente D), jeweils bezogen auf die Gesamtmasse des bevorzugt amorphen Polyurethanharnstoffs. In einer weiteren bevorzugten Ausführungsform wird Komponente D) zur Herstellung des bevorzugt amorphen Polyurethanharnstoffs nicht verwendet.

Als Komponente E) können wahlweise Polyole, insbesondere nicht-polymere Polyole, des genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

In einer bevorzugten Ausführungsform der Erfindung enthält der erfindungsgemäß verwendete bevorzugt amorphen Polyurethanharnstoff ≤ 10 Gew.-% der Komponente E), bevorzugt ≤ 5 Gew.-% der Komponente E), jeweils bezogen auf die Gesamtmasse des bevorzugt amorphen Polyurethanharnstoffs. Bevorzugt beinhaltet der bevorzugt amorphen Polyurethanharnstoff die Komponente E) in einem Bereich von 0,1 bis 10 Gew.-%, bevorzugt in einem Bereich von 0,2 bis 8 Gew.-%, bevorzugt in einem Bereich von 0,1 bis 5 Gew.-%, bezogen jeweils auf die Gesamtmasse des bevorzugt amorphen Polyurethanharnstoffs. In einer weiteren bevorzugten Ausführungsform wird Komponente E) zur Herstellung des bevorzugt amorphen Polyurethanharnstoffs nicht verwendet.

In einer bevorzugten Ausführungsform wird zur Herstellung des erfindungsgemäß verwendeten bevorzugt amorphen Polyurethanharnstoffs ≥ 0,5 und ≤ 20 Gew.-% der Summe der Komponenten C1) und gegebenenfalls E) und besonders bevorzugt ≥ 1 und ≤ 15 Gew.-% der Summe der Komponenten C1) und gegebenenfalls E), jeweils bezogen auf die Gesamtmasse des bevorzugt amorphen Polyurethanharnstoffs, eingesetzt.

Als Komponente F) können weitere polymere Polyole, welche unterschiedlich sind von B) eingesetzt werden.

Beispiele sind polymere Polyole, die nicht unter die Definition von B) fallen, weil sie keine Polyetherpolyole sind - beispielsweise die in der Polyurethanlacktechnologie an sich bekannten Polyesterpolyole, Polyacrylatpolyole, Polyurethanpolyole, Polycarbonatpolyole, Polyesterpolyacrylatpolyole, Polyurethanpolyacrylatpolyole, Polyurethanpolyesterpolyole, Polyurethanpolycarbonatpolyole und Polyesterpolycarbonatpolyole.

Bevorzugt handelt es sich bei der Komponente F) nicht um polymere Polyole die Estergruppen aufweisen, insbesondere nicht um Polyesterpolyole.

Die Komponenten B) und F) enthalten erfindungsgemäß zusammen ≤ 30 Gew.-%, bevorzugt ≤ 10 Gew.-%, und besonders bevorzugt ≤ 5 Gew.-%, an Komponente F), bezogen auf die Gesamtmasse der Komponenten B) und F). Ganz besonders bevorzugt wird die Komponente F) zur Herstellung des bevorzugt amorphen Polyurethanharnstoffs nicht eingesetzt.

In einer bevorzugten Ausführungsform wird zur Herstellung des erfindungsgemäß verwendeten bevorzugt amorphen Polyurethanharnstoffs ≥ 55 und ≤ 90 Gew.-% der Summe der Komponenten B) und gegebenenfalls F) und besonders bevorzugt ≥ 60 und ≤ 85 Gew.-% der Summe der Komponenten B) und gegebenenfalls F), jeweils bezogen auf die Gesamtmasse des bevorzugt amorphen Polyurethanharnstoffs, eingesetzt.

Bei der Komponente G) handelt es sich bevorzugt um Verbindungen, die genau eine isocyanatreaktive Gruppe aufweisen, oder um Verbindungen, die mehr als eine isocyanatreaktive Gruppe aufweisen, wobei nur eine der isocyanatreaktiven Gruppen unter den gewählten Umsetzungsbedingungen mit den in der Reaktionsmischung vorhandenen Isocyanatgruppen reagiert.

Bei den isocyanatreaktiven Gruppen der Komponente G) kann es sich dabei um jede funktionelle Gruppe handeln, die mit einer Isocyanatgruppe reagieren kann, wie beispielsweise Hydroxygruppen, Thiolgruppen oder primäre und sekundäre Aminogruppen.

Isocyanatreaktive Gruppen im Sinne der Erfindung sind dabei insbesondere bevorzugt primären oder sekundäre Aminogruppen, die mit Isocyanatgruppen unter Bildung von Harnstoffgruppen reagieren. Neben der Aminogruppe können Verbindungen der Komponente G) auch andere prinzipiell isocyanatreaktive Gruppen wie beispielsweise auch OH-Gruppen aufweisen, wobei nur eine der isocyanat reaktiven Gruppen unter den gewählten Umsetzungsbedingungen mit den in der Reaktionsmischung vorhandenen Isocyanatgruppen reagiert. Dies kann beispielsweise durch Reaktion entsprechender Aminoalkohole bei relativ niedrigen Temperaturen erfolgen, beispielsweise bei 0 bis 60°C, bevorzugt bei 20 bis 40°C. Bevorzugt wird dabei in Abwesenheit von Katalysatoren gearbeitet, welche die Reaktion von Isocyanatgruppen mit Alkoholgruppen katalysieren würden.

Beispiele für geeignete Verbindungen der Komponente G) sind primäre/sekundäre Amine, wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Diethanolamin, 3-Amino-1-methylaminopropan, 3-Amino-1-ethylaminopropan, 3-Amino-1-cyclohexylaminopropan, 3-Amino-1- methylaminobutan, Ethanolamin, 3-Aminopropanol oder Neopentanolamin.

Geeignete monofunktionellen Verbindungen sind auch Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

In einer bevorzugten Ausführungsform wird zur Herstellung des erfindungsgemäß verwendeten bevorzugt amorphen Polyurethanharnstoffs ≥ 0,1 und ≤ 20 Gew.-% der Komponente G) und besonders bevorzugt ≥ 0,3 und ≤ 10 Gew.-% der Komponente G), jeweils bezogen auf die Gesamtmasse des bevorzugt amorphen Polyurethanharnstoffs, eingesetzt.

In einer besonders bevorzugten Ausführungsform der Erfindung wird die Komponente H) eingesetzt und das molare Verhältnis von Komponente G) zu Komponente H) beträgt bevorzugt 5:1 bis 1:5, besonders bevorzugt 1,5:1 bis 1:4 und ganz besonders bevorzugt 1:1 bis 1:3.

In einer bevorzugten Ausführungsform werden zur Herstellung der erfindungsgemäß verwendeten bevorzugt amorphen Polyurethanharnstoffe die Komponenten A) bis H) in den folgenden Mengen eingesetzt, wobei sich die Einzelmengen stets zu 100 Gew.-% addieren:
5 bis 40 Gew.-% Komponente A),
55 bis 90 Gew.-% Summe der Komponenten B) und gegebenenfalls F),
0,5 bis 20 Gew.-% Summe der Komponenten C1) und gegebenenfalls E),
0,1 bis 10 Gew.-% Komponente C2),
0 bis 20 Gew.-% Komponente D),
0,1 bis 20 Gew.-% der Komponente G) und
0 bis 10 Gew.-% Komponente H).

In einer besonders bevorzugten Ausführungsform werden zur Herstellung der erfindungsgemäß verwendeten bevorzugt amorphen Polyurethanharnstoffe die Komponenten A) bis H) in den folgenden Mengen eingesetzt, wobei sich die Einzelmengen stets zu 100 Gew.-% aufaddieren:
10 bis 35 Gew.-% Komponente A),
60 bis 85 Gew.-% Summe der Komponenten B) und gegebenenfalls F),
1 bis 15 Gew.-% Summe der Komponenten C1) und gegebenenfalls E),
0,5 bis 4 Gew.-% Komponente C2),
0 bis 10 Gew.-% Komponente D),
0,3 bis 10 Gew.-% der Komponente G) und
0,1 bis 3 Gew.-% Komponente H).

In einer bevorzugten Ausführungsform der Erfindung umfasst der bevorzugt amorphe Klebstoff einen bevorzugt amorphen Polyurethanharnstoff, der erhältlich ist, durch Umsetzung von mindestens
A) einer aliphatischen, cycloaliphatischen oder araliphatischen Polyisocyanat-Komponente mit einer mittleren Isocyanatfunktionalität von ≥ 1,8 und ≤ 2,6, welche ausgewählt ist aus HDI, IPDI und/oder H12-MDI oder deren Modifikationsprodukten,
B) einer polymeren Polyether-Polyol-Komponente, welche bevorzugt aus Poly(propylenglykol)polyetherpolyole besteht,
C) einer aminofunktionellen Kettenverlängerer-Komponente mit mindestens 2 isocyanatreaktiven primären und/oder sekundären Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls weiteren polymeren Polyolen, welche unterschiedlich sind von B),
G) gegebenenfalls einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und
H) gegebenenfalls einer aliphatischen, cycloaliphatischen oder araliphatischen Polyisocyanat-Komponente mit einer mittleren Isocyanatfunktionalität von > 2,6 und ≤ 4, wobei die Komponente H) aus einem aliphatischen oder cycloaliphatischen Polyisocyanat-Oligomer mit Isocyanurat-, Urethan-, Allophanat-, Biuret-, Iminooxadiazindion- oder Oxadiazintrionstruktur besteht,
wobei die Komponenten B) und F) zusammen ≤ 30 Gew.-% an Komponente F), bezogen auf die Gesamtmasse der Komponenten B) und F) enthalten.

In einer besonders bevorzugten Ausführungsform der Erfindung umfasst der bevorzugt amorphe Klebstoff einen bevorzugt amorphen Polyurethanharnstoff, der erhältlich ist, durch Umsetzung von mindestens
A) einer aliphatischen Polyisocyanat-Komponente, wobei es sich um ein Gemisch aus IPDI und HDI handelt,
B) einer polymeren Polyether-Polyol-Komponente, welche ein Gemisch aus wenigstens zwei Poly(propylenglykol)polyetherpolyole ist und wobei sich die Poly(propylenglykol)polyetherpolyole in ihren zahlenmittleren Molekulargewichten unterscheiden,
C) einer aminofunktionellen Kettenverlängerer-Komponente mit 2 isocyanatreaktiven primären und/oder sekundären Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
D) gegebenenfalls weiteren hydrophilierenden Komponenten, die unterschiedlich sind von C2), wobei es sich um nichtionisch hydrophilierende Komponenten D1) handelt,
E) gegebenenfalls hydroxyfunktionellen Verbindungen mit einem Molekulargewicht von 62 bis 399 mol/g,
F) gegebenenfalls weitere polymere Polyole, welche unterschiedlich sind von B),
G) gegebenenfalls einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert, wobei es sich bei der isocyanat reaktiven Gruppe um eine primäre und/oder sekundäre Amino- und/oder Hydroxygruppe handelt und
H) gegebenenfalls einer aliphatischen Polyisocyanat-Komponente mit einer mittleren Isocyanatfunktionalität von > 2,6 und ≤ 4, wobei die Komponente H) aus einem aliphatischen oder cycloaliphatischen Polyisocyanat-Oligomer mit Isocyanurat-, Urethan-, Allophanat-, Biuret-, Iminooxadiazindion- oder Oxadiazintrionstruktur, basierend auf HDI, IPDI und/oder H12-MDI besteht,
wobei die Komponenten B) und F) zusammen ≤ 30 Gew.-% an Komponente F), bezogen auf die Gesamtmasse der Komponenten B) und F) enthalten.

Bevorzugt ist der erfindungsgemäß verwendete bevorzugt amorphe Polyurethanharnstoff erhältlich durch Umsetzung ausschließlich der Komponenten A) bis H). Es werden dann keine weiteren Komponenten zur Herstellung des bevorzugt amorphen Polyurethanharnstoffs eingesetzt.

Die erfindungsgemäß verwendeten bevorzugt amorphen Polyurethanharnstoffe sind bevorzugt lineare Moleküle, können alternativ jedoch auch verzweigt sein.

Das zahlenmittlere Molekulargewicht der bevorzugt verwendeten bevorzugt amorphen Polyurethanharnstoffe beträgt bevorzugt von ≥ 2000 bis ≤ 300000 g/mol, bevorzugt von ≥ 5000 bis ≤ 150000 g/mol, oder bevorzugt von ≥10000 bis ≤ 100000 g/mol.

Der zur Herstellung des Klebstoffes eingesetzte bevorzugt amorphe Polyurethanharnstoff liegt bevorzugt in einem physiologisch akzeptablen Medium vor. Besonders bevorzugt handelt es sich bei dem Medium um Wasser und ganz besonders bevorzugt liegt der bevorzugt amorphen Polyurethanharnstoff als wässrige Dispersion vor, die im Wesentlichen keine weiteren Lösungsmittel aufweist. Unter im Wesentlichen keine weiteren Lösungsmittel wird gemäß der Erfindung verstanden, dass weniger als 2 Gew.-%, bevorzugt weniger als 1,5 Gew.-%, oder bevorzugt weniger als 1 Gew.-%, bezogen auf das Gesamtgewicht der Polyurethandispersion, keine weiteren Lösungsmittel in der Polyurethandispersion vorhanden sind, insbesondere keine organischen Lösungsmittel wie beispielsweise Aceton. Wasser bildet im Allgemeinen, neben wahlweise vorhandenen anderen flüssigen Medien, wie beispielsweise Lösungsmitteln, den Hauptbestandteil (> 50 Gew.-%) des Dispergiermediums, bezogen auf die Gesamtmenge des flüssigen Dispergiermediums, gegebenenfalls auch das alleinige flüssige Dispergiermedium. Bevorzugt ist der verwendete bevorzugt amorphen Polyurethanharnstoff daher in Wasser dispergierbar, was im Rahmen dieser Erfindung bedeutet, dass der bevorzugt amorphen Polyurethanharnstoff bei 23°C eine sedimentationsstabile Dispersion in Wasser, insbesondere entionisiertem Wasser, bilden kann.

In einer bevorzugten Ausführungsform des Klebstoffs sind die verwendeten bevorzugt amorphen Polyurethanharnstoffe erhältlich, indem isocyanatfunktionelle Polyurethanpräpolymere a) aus den Komponenten A), B) und gegebenenfalls D) und/oder C2), sowie gegebenenfalls den Verbindungen E) und/oder H) hergestellt werden (Schritt a) und deren freie NCO-Gruppen anschließend ganz oder teilweise mit der aminofunktionellen Kettenverlängerer-Komponente C), sowie der Komponente G) und gegebenenfalls der Komponente D) und H) umgesetzt werden (Schritt b)).

Wobei wenn die Komponente H) erst in Schritt b) eingesetzt wird, diese bevorzugt vor der Zugabe der Komponente C) zugegeben und mit dem Prepolymer a) umgesetzt wird.

In einer bevorzugten Ausführungsform der Erfindung erfolgt im Schritt b) die Umsetzung mit einem Diamin oder mehreren Diaminen (Komponente C) unter Kettenverlängerung wobei außerdem die monofunktionelle Komponente G) als Kettenabbrecher zur Molekulargewichtssteuerung zugesetzt wird.

Die Komponenten A) bis H) sind dabei wie oben angegeben definiert. Es gelten auch die oben genannten bevorzugten Ausführungsformen.

Bevorzugt wird im Schritt b) der Umsetzung des Präpolymers a) zur Herstellung des bevorzugt amorphen Polyurethanharnstoffs eine Mischung aus Komponenten C1), C2) und G) zur Umsetzung gebracht. Durch die Verwendung der Komponente C1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten C1), C2) und G) lässt sich eine optimale Balance zwischen Hydrophilie und Kettenlänge einstellen.

Bevorzugt weist das erfindungsgemäß verwendete Polyurethanpräpolymer a) endständige Isocyanatgruppen auf, d.h. die Isocyanatgruppen liegen an den Kettenenden des Präpolymers. Besonders bevorzugt weisen sämtliche Kettenenden des Präpolymers Isocyanatgruppen auf.

Über die hydrophilierende Komponente C2) und/oder D) kann die Hydrophilie des Präpolymers gesteuert werden. Daneben spielen für die Hydrophilie des Präpolymers natürlich auch noch weitere Komponenten, speziell auch die Hydrophilie der Komponente B) eine Rolle.

Bevorzugt sind die isocyanatfunktionellen Polyurethanpräpolymere a) wasserunlöslich und nicht in Wasser dispergierbar.

Im Rahmen der Erfindung bedeutet der Begriff "wasserunlösliches, nicht wasserdispergierbares Polyurethanpräpolymer" insbesondere, dass die Wasserlöslichkeit des erfindungsgemäß verwendeten Präpolymers bei 23°C weniger als 10 g / Liter, bevorzugter weniger als 5 g / Liter beträgt und das Präpolymer bei 23° keine sedimentationsstabile Dispersion in Wasser, insbesondere entionisiertem Wasser, ergibt. Mit anderen Worten setzt sich das Präpolymer, beim Versuch es in Wasser zu dispergieren, ab. Die Wasserunlöslichkeit bzw. fehlende Dispergierbarkeit in Wasser bezieht sich auf entionisiertes Wasser ohne Zusatz von Tensiden.

Weiterhin weist das verwendete Polyurethanpräpolymer a) bevorzugt im Wesentlichen weder ionische noch ionogene (zur Bildung von ionischen Gruppen befähigte) Gruppen auf. Im Rahmen der vorliegenden Erfindung bedeutet dies, dass der Anteil der ionischen und/oder ionogenen Gruppen, wie insbesondere anionischer Gruppen, wie Carboxylat oder Sulfonat, oder kationischer Gruppen weniger beträgt als 15 Milliequivalente pro 100 g Polyurethanpräpolymer a1), bevorzugt weniger als 5 Milliequivalente, besonders bevorzugt weniger als 1 Milliequivalent und ganz besonders bevorzugt weniger als 0,1 Milliequivalente pro 100 g Polyurethanpräpolymer a).

Im Falle saurer ionischer und/oder ionogener Gruppen beträgt die Säurezahl des Präpolymers zweckmäßig unter 30 mg KOH/g Präpolymer, bevorzugt unter 10 mg KOH/g Präpolymer. Die Säurezahl gibt die Masse Kaliumhydroxid in mg an, die zur Neutralisation von 1 g der zu untersuchenden Probe erforderlich ist (Messung nach DIN EN ISO 211). Die neutralisierten Säuren, also die entsprechenden Salze weisen naturgemäß keine oder eine reduzierte Säurezahl auf. Hier ist erfindungsgemäß die Säurezahl der korrespondierenden freien Säure entscheidend.

Dabei sind die wasserunlöslichen, nicht wasserdispergierbaren, isocyanatfunktionellen Polyurethanpräpolymeren a) bevorzugt ausschließlich erhältlich aus den Komponenten A), B) und gegebenenfalls D), E) und/oder H).

Die Komponenten sind dabei wie oben angegeben definiert. Es gelten auch die oben genannten bevorzugten Ausführungsformen.

Es werden folglich bevorzugt in dieser Ausführungsform keine ionisch hydrophilierenden Komponenten C2) oder auch D2) zur Herstellung des Präpolymers a) eingesetzt. Auch wird die Komponente G) in diesem Schritt nicht zugesetzt. Die Hydrophilierungsmittel D1) werden bevorzugt in solchen Mengen eingesetzt, dass das Präpolymer dennoch wasserunlöslich und nicht wasserdispergiebar ist. Besonders bevorzugt werden ≤ 10 Gew.-% der Komponente D1), ganz besonders bevorzugt ≤ 5 Gew.-% und weiterhin bevorzugt ≤ 2 Gew.-% der Komponente D1), jeweils bezogen auf die Gesamtmasse des bevorzugt amorphen Polyurethanharnstoffs, eingesetzt. Weiterhin bevorzugt wird die Komponente D1) zur Herstellung des Präpolymers a) nicht eingesetzt.

In einer bevorzugten Ausführungsform der Erfindung weist die Komponente B) weder ionische noch ionogene Gruppen auf. Weiterhin werden bei dieser Ausführungsform der Erfindung als Komponente B) bevorzugt nur Polyetherpolyole, insbesondere Polyalkylenoxidether eingesetzt, die ≤ 10 mol-% und bezogen auf alle enthaltenen Alkylenoxideinheiten an Ethylenoxideinheiten und bevorzugt keine Ethylenoxideinheiten enthalten.

Die in dieser Ausführungsform der Erfindung bevorzugt verwendeten bevorzugt amorphen Polyurethanharnstoffe weisen folglich ionische oder ionogene Gruppen auf, bevorzugt anionische Gruppen auf, diese anionischen Gruppen werden dabei in die verwendeten bevorzugt amorphen Polyurethanharnstoffe über die in Schritt b) eingesetzte hydrophilierende Komponente C2) eingeführt. Die verwendeten bevorzugt amorphen Polyurethanharnstoffe weisen gegebenenfalls zusätzlich nichtionische Komponenten zu Hydrophilierung auf.

Besonders bevorzugt sind in den verwendeten bevorzugt amorphen Polyurethanharnstoffen zur Hydrophilierung ausschließlich Sulfonatgruppen enthalten, welche in Schritt b) über entsprechende Diamine als Komponente C2) in den bevorzugt amorphen Polyurethanharnstoff eingeführt werden.

In einer alternativen, weniger bevorzugten Ausführungsform der Erfindung sind die zur Herstellung des erfindungsgemäßen bevorzugt amorphen Polyurethanharnstoffs eingesetzten Präpolymere a) wasserlöslich oder wasserdispergierbar. In dieser Ausführungsform werden die hydrophilierende Komponente D) und/oder C2) bei der Herstellung des Präpolymers a) in einer Menge eingesetzt, die ausreichend ist, damit das Präpolymer wasserlöslich oder wasserdispergierbar ist. Das Präpolymer a) weist dabei bevorzugt ionische oder ionogene Gruppen auf.

Geeignete hydrophilierende Komponenten D) und C2) sind für diese Ausführungsform der Erfindung die oben für D) und C2) genannten Verbindungen. Bevorzugt werden als hydrophilierende Komponenten wenigstens die oben unter D1) und/oder C2) genannten Verbindungen eingesetzt.

Die zur Herstellung des erfindungsgemäßen Klebstoffes verwendeten bevorzugt amorphen Polyurethanharnstoffe werden bevorzugt vor, während oder nach Schritt b), besonders bevorzugt während oder nach Schritt b) in Wasser dispergiert. So wird eine Dispersion der bevorzugt amorphen Polyurethanharnstoffe erhalten.

Die Herstellung der bevorzugt amorphen Polyurethanharnstoff-Dispersionen kann dabei in einer oder mehreren Stufe/-n in homogener oder bei mehrstufiger Umsetzung, teilweise in disperser Phase durchgeführt werden. Nach Herstellung des Präpolymers a) erfolgt bevorzugt ein Dispergier-, Emulgier- oder Lösungsschritt. Im Anschluss erfolgt gegebenenfalls eine weitere Polyaddition oder Modifikation in disperser Phase. Dabei wird jeweils das für das entsprechende Präpolymer geeignete Löse- oder Dispergiermittel wie beispielsweise Wasser oder Aceton oder Mischungen daraus gewählt.

Dabei können alle aus dem Stand der Technik bekannten Verfahren wie z. B. Präpolymer-Mischverfahren, Acetonverfahren oder Schmelzdispergierverfahren verwendet werden. Bevorzugt wird das Aceton-Verfahren angewandt.

Für die Herstellung nach dem Aceton-Verfahren werden üblicherweise die Bestandteile B), gegebenenfalls D) und E) und die Polyisocyanatkomp,onente A) gegebenfalls in Kombination mit der Komponente H) zur Herstellung eines isocyanatfunktionellen Polyurethanpräpolymers ganz oder teilweise vorgelegt und gegebenenfalls mit einem mit Wasser mischbaren aber gegenüber Isocyanat-gruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, jedoch nicht bevorzugt.

Anschließend können die gegebenenfalls zu Beginn der Reaktion noch nicht zugegebenen Bestandteile von A), B) und gegebenenfalls H), D) und E) zudosiert werden.

Bei der Herstellung des Polyurethan-Präpolymeren aus A), B) und gegebenenfalls H), D) und E) beträgt das Stoffmengenverhältnis von Isocyanat-Gruppen zu isocyanatreaktiven Gruppen bevorzugt 1,05 bis 3,5, besonders bevorzugt 1,1 bis 3,0 und ganz besonders bevorzugt 1,1 bis 2,5.

Die Umsetzung der Komponenten A), B) und gegebenenfalls H), D) und E) zum Präpolymer kann teilweise oder vollständig, bevorzugt aber vollständig erfolgen. Es können so Polyurethanpräpolymere, die freie Isocyanatgruppen enthalten, in Substanz oder in Lösung erhalten werden.

Sollten ionogene Gruppen, wie beispielsweise Carboxylgruppen, im Präpolymer vorliegen können diese in einem weiteren Schritt durch Neutralisation in ionische Gruppen überführt werden.

Im Neutralisationsschritt zur teilweisen oder vollständigen Überführung potentiell anionischer Gruppen in anionische Gruppen können Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder ganz besonders bevorzugt Alkalimetallbasen wie die entsprechenden Hydroxide eingesetzt werden.

Als Neutralisationsmittel sind bevorzugt anorganische Basen, wie wässrige Ammoniaklösung oder Natrium- bzw. Kaliumhydroxid einsetzbar, besonders bevorzugt sind Natriumhydroxid und Kaliumhydroxid.

Die Stoffmenge der Basen beträgt bevorzugt 50 und 125 mol%, besonders bevorzugt zwischen 70 und 100 mol% der Stoffmenge der zu neutralisierenden Säuregruppen. Die Neutralisation kann, auch gleichzeitig mit der Dispergierung erfolgen, in dem das Dispergierwasser bereits das Neutralisationsmittel enthält.

Im Anschluss an die Neutralisation wird in einem weiteren Verfahrensschritt, falls noch nicht oder nur teilweise geschehen, das erhaltene Präpolymer mit Hilfe von aliphatischen Ketonen wie Aceton oder 2-Butanon gelöst.

Bei der Kettenverlängerung/-terminierung in Stufe b) werden die Komponenten C), G) und gegebenenfalls D) mit den noch verbliebenen Isocyanatgruppen des Präpolymers umgesetzt. Bevorzugt wird die Kettenverlängerung/-terminierung vor der Dispergierung in Wasser durchgeführt.

Geeignete Komponenten C) zur Kettenverlängerung sowie G) zum Kettenabbruch sind bereits oben aufgeführt. Es gelten analog auch die oben genannten bevorzugten Ausführungsformen.

Werden zur Kettenverlängerung anionische Hydrophilierungsmittel entsprechend der Definition C2) mit NH₂- oder NH-Gruppen eingesetzt, erfolgt die Kettenverlängerung der Präpolymere in Schritt b) bevorzugt vor der Dispergierung in Wasser.

Das Äquivalentverhältnis von NCO-reaktiven Gruppen der zur Kettenverlängerung und Kettenterminierung eingesetzten Verbindungen zu freien NCO-Gruppen des Präpolymers, liegt im allgemeinen zwischen 40 und 150 %, bevorzugt zwischen 50 und 110 %, besonders bevorzugt zwischen 60 und 100 %.

Die Komponenten C1), C2) und G), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel in Schritt b) mit verwendet werden, so beträgt der jeweilige Verdünnungsmittelgehalt in den eingesetzten Komponenten C1), C2) und G) bevorzugt 40 bis 95 Gew.-%.

Die Dispergierung erfolgt bevorzugt im Anschluss an die Kettenverlängerung und Kettenterminierung. Dazu wird das gelöste (beispielsweise in Aceton) und mit dem Aminen umgesetzte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste Polyurethanpolymer gegeben.

Das in den Dispersionen nach dem Dispergierschritt noch enthaltene Lösemittel wird üblicherweise anschließend durch Destillation entfernt. Eine Entfernung bereits während der Dispergierung ist ebenfalls möglich.

Die erhaltenen wässrigen Polyurethanharnstoffdispersionen weisen bevorzugt einen Gehalt an flüchtigen organischen Verbindungen (VOC), wie beispielsweise flüchtige organische Lösemitteln, von weniger als 10 Gew.-%, bevorzugter von weniger als 3 Gew.-%, noch bevorzugter von weniger als 1 Gew.-% bezogen auf die auf wässrige Polyurethanharnstoffdispersion auf. VOCs im Sinne dieser Erfindung sind insbesondere organische Verbindung mit einem Anfangssiedepunkt von höchstens 250 °C bei einem Standarddruck von 101,3 kPa.

Die Bestimmung des Gehalts an flüchtigen organischen Verbindungen (VOC) erfolgt im Rahmen der vorliegenden Erfindung insbesondere durch gaschromatographische Analyse.

Der pH-Wert der verwendeten wässrigen Polyurethan-Dispersionen beträgt typischerweise weniger als 8,0, bevorzugt weniger als 7,5 und liegt besonders bevorzugt zwischen 5,5 und 7,5.

Um eine gute Sedimentationsstabilität zu erreichen, liegt die zahlenmittlere Teilchengröße der speziellen Polyurethanharnstoff-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser (Gerät: Malvern Zetasizer 1000, Malvern Inst. Limited).

Der Feststoffgehalt der Polyurethanharnstoff-Dispersionen beträgt bevorzugt 10 bis 70 Gew.-%, besonders bevorzugt 20 bis 60 Gew.-%und ganz besonders bevorzugt 40 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Bevorzugt weisen diese Polyurethanharnstoff-Dispersionen weniger als 5 Gew.-%, besonders bevorzugt weniger als 0,2 Gew.-%, bezogen auf die Masse der Dispersionen, an ungebundenen organischen Aminen auf.

Die zur Herstellung des Klebstoffs eingesetzte Polyurethanharnstoff-Dispersionen weisen bei 23°C bei einer konstanten Scherrate von 10 s⁻¹ bevorzugt eine Viskosität von ≥ 1 und ≤ 10000 mPa s, besonders bevorzugt von ≥ 10 und ≤ 5000 mPa s und ganz besonders bevorzugt von ≥ 100 und ≤ 4000 mPa s auf. Die Viskosität wird dabei wie im Methodenteil beschrieben bestimmt.

Der für die Herstellung des Klebstoffs verwendete Polyurethanharnstoff ist bevorzugt amorph. In einer bevorzugten Ausführungsform des Klebstoffs weist der verwendete Polyurethanharnstoff einen T_{g} von ≤ - 25 °C, oder bevorzugt von ≤ - 50 °C, oder bevorzugt von ≤ - 70 °C auf.

Amorph bedeutet im Sinne dieser Erfindung, dass der Polyurethanharnstoff im in der im folgenden ausgeführten Messmethode genannten Temperaturbereich keine oder nur so geringe kristalline Anteile ausbildet, dass mittels der beschriebenen DSC Messungen nur ein oder mehrere Glasübergangspunkte T_{g}, aber keine Schmelzbereiche mit einer Schmelzenthalpie ≥ 20 J/g in dem genannten Temperaturbereich gefunden werden können.

Die Glasübergangstemperatur T_{g} wird im Rahmen dieser Erfindung mittels dynamischer Differenzkalorimetrie in Anlehnung an die DIN EN 61006, Verfahren A, bestimmt, wobei ein DSC Gerät verwendet wird, das zur Bestimmung von T_{g} mit Indium und Blei kalibriert ist und wobei drei unmittelbar aufeinander folgende Durchläufe von Aufheizungen von -100°C bis +150°C, mit einer Heizrate von 20 K/min, mit jeweils anschließender Abkühlung mit einer Kühlrate von 320 K/min vorgenommen und die dritte Aufheizkurve zur Bestimmung der Werte verwendet wird und wobei als T_{g} die Temperatur bei der halben Höhe einer Glasübergangsstufe bestimmt wird.

Sollte der Polyurethanharnstoff in Form einer Dispersion vorliegen, wird bei der Probenpräparation für die DSC Messungen besonders vorgegangen. Bei der Bestimmung der Glasübergangstemperatur T_{g} von Dispersionen mittels DSC kann die T_{g} des Polymers durch die kalorischen Effekte des Dispergens (Wasser, Neutralisationsmittel, Emulgatoren, Colöser, etc.) maskiert bzw. wegen der Mischbarkeit mit dem Polymer deutlich abgesenkt werden. Daher wird das Dispergens vor der DSC-Messung bevorzugt durch geeignete Trocknung zunächst vollständig entfernt, denn auch geringe Restmengen Dispergens wirken als Weichmacher und können die Glastemperatur dadurch absenken. Die Dispersion wird daher bevorzugt mit 200 µm Nassschichtdicke (NSD) mit einem Kastenrakel aus Edelstahl auf eine Glasplatte gerakelt, abgelüftet und dann für zwei Tage in einer Trockenbox bei RT und 0% relativer Luftfeuchtigkeit (rF) schonend getrocknet. Nach dieser Probenpräparation kann in der ersten Aufheizung der DSC Messung noch ein breiter endothermer Verdampfungsbereich von Restfeuchte im Filmauftreten. Um die bestimmten Werte möglichst von solchen Einflüssen frei zu erhalten, wird daher die dritte Aufheizkurve ausgewertet.

Die Komponente I) ist bevorzugt ausgewählt aus der Gruppe niedermolekularer, aliphatischer Diisocyanate einer Molmasse von 140 bis 278 g/mol und daraus herstellbare Polyisocyanate mit einer Isocyanatfunktionalität von 2 bis 3,6, oder bevorzugt von 2 bis 3 oder Mischungen aus mindestens zwei hiervon.

Die Komponente J) ist bevorzugt ein monofunktionelles Polyalkylenoxid einer OH-Zahl von 10 bis 250, oder bevorzugt von 15 bis 220, oder bevorzugt von 20 bis 200. Bevorzugt ist das Polyalkylenoxid ausgewählt aus der Gruppe bestehend aus Ethylenoxid, Propylenoxid, Butylenoxid, Pentylenoxid oder einer Mischung aus mindestens zwei hiervon. Bevorzugt weist die Polyalkylenoxid -Komponente einen Ethylenoxidanteil von 50 bis 100 mol% bezogen auf die Gesamtmenge der enthaltenen Oxyalkylengruppen auf.

Bei der Herstellung der hydrophilen Polyisocyanate K2. wird das Verhältnis der monofunktionellen Polyalkylenoxide J) zu den niedermolekularen, aliphatischen Diisocyanaten I) typischerweise so eingestellt, dass auf 1 Mol OH-Gruppen der monofunktionellen Polyalkylenoxide 1,25 bis 20 Mol, bevorzugt 2 bis 15 Mol, oder bevorzugt 5 bis 13 Mol NCO-Gruppen des niedermolekularen, aliphatischen Diisocyanats I) kommen. Anschließend erfolgt bevorzugt die Allophanatisierung bzw. Biurethisierung und/oder Isocyanuratbildung bzw. Uretdionbildung. Werden die Polyalkylenoxide J) über Urethangruppen an die aliphatischen Diisocyanate I) gebunden, findet bevorzugt im Anschluss eine Allophanatisierung statt. Weiterhin ist bevorzugt, dass Isocyanurat-Struktureinheiten gebildet werden.

Eine bevorzugte alternative Herstellung der hydrophilen Polyisocyanate K2. erfolgt typischerweise durch Umsetzung von 1 Mol OH-Gruppen der monofunktionellen Polyalkylenoxid-Komponente J) mit 1,25 bis 20 Mol, bevorzugt mit 2 bis 15 Mol und besonders bevorzugt 5 bis 13 NCO-Gruppen eines Polyisocyanates I) mit einer Isocyanatfunktionalität von 2 bis 3,6, basierend auf aliphatischen Diisocyanaten. Beispielhaft für derartige Polyisocyanate I) sind Biuret-Strukturen, Isocyanurate bzw. Uretdione basierend auf aliphatischen Diisocyanaten. Dabei werden das Polyisocyanat I) und das Polyalkylenoxid J) bevorzugt über eine Urethangruppe bzw. eine Harnstoffgruppe miteinander verknüpft, wobei besonders die Verknüpfung über Urethangruppen bevorzugt ist.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt 60 bis 100 °C.

Wurde mit überschüssigem niedermolekularen Diisocyanat gearbeitet, erfolgt anschließend die Entfernung des Überschusses an niedermolekularem, aliphatischen Diisocyanat bevorzugt durch Dünnschichtdestillation.

Vor, während und/oder nach der Reaktion oder der destillativen Abtrennung des Diisocyanatüberschusses können saure oder alkylierende Stabilisatoren, wie Benzoylchlorid, Isophthaloyl¬chlorid, Methyltosylat, Chlorpropionsäure, HCl oder Antioxidantien, wie Di-tert-butylkresol oder Tocopherol zugesetzt werden.

Der NCO-Gehalt der hydrophilen Polyisocyanate K2. beträgt bevorzugt 0,3 bis 23 Gew.-%, besonders bevorzugt 2 bis 21 Gew.-% und ganz besonders bevorzugt 3 bis 18 Gew.-%.

Beispiele für niedermolekulare, aliphatische Diisocyanate der Komponente I) sind Hexamethylendiisocyanat (HDI), Isophorondiisocyanat (IPDI), Butylendiisocyanat (BDI), Pentamethylendiisocyanat (PDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI), Pentamethylendiisocyanat (PDI), und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat.

Beispiele für höhermolekulare Polyisocyanate I) sind Polyisocyanate mit einer Isocyanatfunktionalität von 2 bis 3,6 mit Isocyanurat-, Urethan , Allophanat , Biuret , Iminooxadiazintrion-, Oxadia¬zintrion- und/oder Uret¬dion¬gruppen auf Basis der im vorstehenden Abschnitt genannten aliphatischen und/oder cycloaliphatischen Diisocyanate.

Bevorzugt werden als Komponente I) höhermolekulare Verbindungen mit Biuret-, Iminooxadiazindion,- Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die monofunktionellen Polyalkylenoxiden J) weisen eine OH-Zahl von 15 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Unter monofunktionellen Polyalkylenoxiden im Sinne der Erfindung sind Verbindungen zu verstehen, die nur eine isocyanatreaktive Gruppe, d.h. eine Gruppe, die mit einer NCO-Gruppe reagieren kann, aufweisen.

Die Herstellung von Polyalkylenoxiden J) durch Alkoxylierung geeig¬neter Startermoleküle ist literaturbekannt (z.B. Ull¬manns Encyclopädie der technischen Chemie, 4. Auflage, Band 19, Verlag Chemie, Weinheim S. 31-38). Geeignete Startermoleküle sind insbesondere gesättigte Monoalkohole wie Metha¬nol, Ethanol, n-Propanol, Isopropanol, n-Butanol, Isobutanol, sec-Butanol, Diethylen¬glykolmonobutylether sowie aromatische Alkohole wie Phenol oder Monoamine wie Diethyl¬amin. Bevor¬zugte Startermoleküle sind gesättigte Monoalkohole der vorstehend genannten Art. Besonders bevorzugt werden Diethylen¬glykolmonobutylether oder n-Butanol als Star¬termoleküle verwendet.

Die monofunktionellen Polyalkylenoxide J) besitzen typischerweise zahlenmittlere Molekulargewichte von 220 bis 3700 g/mol, bevorzugt von 250 bis 2800 g/mol, oder bevorzugt von 300 bis 2000 g/mol.

Die monofunktionellen Polyalkylenoxide J) besitzen bevorzugt eine OH-Gruppe als isocyanatreaktive Gruppe.

In einer bevorzugten Ausführungsform des Klebstoffes ist:
I) ausgewählt aus der Gruppe bestehend aus
   I1.) niedermolekularen, aliphatischen Diisocyanaten einer Molmasse von 140 bis 278 g/mol,
   I2.) aus I1.) herstellbare Polyisocyanate mit einer Isocyanatfunktionalität von 2 bis 3,6,
   I3.) einer Kombination aus I1.) und I2.),
J) ausgewählt aus der Gruppe bestehend aus
   J1.) einem monofunktionellen Polyalkylenoxid einer OH-Zahl von 10 bis 250,
   J2.) Ethylenoxid, Propylenoxid, Butylenoxid, Pentylenoxid oder einer Mischung aus mindestens zwei hiervon,
   J3.) einem monofunktionellen Polyalkylenoxid mit einem Ethylenoxidanteil von 50 bis 100 mol%, bezogen auf die Gesamtmenge der enthaltenen Oxyalkylengruppen,
   J4.) einer Kombination aus mindestens zwei von J1.) bis J3.).

Typische weitere geeignete Zuschlags- und Hilfsstoffe L) sind Oberflächenadditive wie z.B. Benetzungshilfsmittel, Farbstoffe und/oder Verlaufshilfsmittel.

Die Polyurethanharnstoff-Dispersion kann auch alle weiteren dem Fachmann für die jeweilige Anwendung bekannten Zuschlagstoffe enthalten.

Ein weiterer Gegenstand der Erfindung betrifft eine wässrige Polyurethanharnstoff-Dispersion, enthaltend
(V1) einen amorphen Polyurethanharnstoff, welcher erhältlich ist, durch Umsetzung von mindstens
   A) einer aliphatischen, cycloaliphatischen oder araliphatischen Polyisocyanat-Komponente mit einer mittleren Isocyanatfunktionalität von ≥ 1,8 und ≤ 2,6,
   B) einer polymeren Polyether-Polyol-Komponente,
   C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
   D) gegebenenfalls weiteren hydrophilierenden Komponenten, die unterschiedlich sind von C2),
   E) gegebenenfalls hydroxyfunktionellen Verbindungen mit einem Molekulargewicht von 62 bis 399 mol/g,
   F) gegebenenfalls weitere polymere Polyolen, welche unterschiedlich sind von B)
   G) gegebenenfalls einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und
   H) gegebenenfalls einer aliphatischen, cycloaliphatischen oder araliphatischen Polyisocyanat-Komponente mit einer mittleren Isocyanatfunktionalität von > 2,6 und ≤ 4,
   wobei die Komponenten B) und F) zusammen ≤ 30 Gew.-% an Komponente F), bezogen auf die Gesamtmasse der Komponenten B) und F) enthalten,
   sowie
(V2) ein hydrophiles Polyisocyanat herstellbar aus
   I) einer aliphatischen, cycloaliphatischen oder araliphatischen Polyisocyanat-Komponente mit einer mittleren Isocyanatfunktionalität von bevorzugt ≥ 2,0 und ≤ 3,6
   J) eine polymere, hydrophile und monofunktionelle Polyalkylenoxid--Komponente,
   K) gegebenenfalls weitere hydrophilierende Komponenten, die unterschiedlich sind von J),
   L) gegebenenfalls Zuschlags- und Hilfsstoffen.

Bevorzugt sind die Komponenten A) bis L) die gleichen, wie für die entsprechenden Komponenten aufgeführten Auswahlen für den Klebstoff. Alle im Zusammenhang mit dem Klebstoff aufgeführten Eigenschaften, Mengen, Verhältnisse und Zusammensetzungen bezüglich der Komponenten A) bis L) gelten für die Polyurethanharnstoff-Dispersion und das eingesetzte hydrophile Polyisocyanat ebenfalls.

Ein weiterer Gegenstand der Erfindung betrifft ein Verfahren zur Herstellung eines Schichtaufbaus beinhaltend mindestens eine Klebstoffschicht, umfassend die Schritte:
(I) Mischen eines hydrophilen Polyisocyanats (V2) zu einem Polyurethanharnstoff (V1) unter Erhalt einer erfindungsgemäßen Polyurethanharnstoff-Dispersion,
(II) Aufbringen der Polyurethanharnstoff-Dispersion auf eine erste weitere Schicht, unter Erhalt eines Vorläufers,
(III) thermische Behandlung des Vorläufers aus Schritt (II) bei Temperaturen in einem Bereich von 20 °C bis 200 °C, unter Bildung der Klebstoffschicht.

Die in dem Verfahren hergestellte Klebstoffschicht ist bevorzugt so ausgestaltet wie die zuvor beschriebene Klebstoffschicht. Bevorzugt weist die mit dem erfindungsgemäßen Verfahren hergestellte Klebstoffschicht die gleichen Materialien, Eigenschaften und Ausgestaltungen auf, wie bereits für die erfindungsgemäße Klebstoffschicht beschrieben.

Das Mischen des hydrophilen Polyisocyanats (V2) in die Polyurethanharnstoff-Dispersion (V1) unter Erhalt einer Polyurethanharnstoff-Mischung kann auf jede Art erfolgen, die der Fachmann hierfür auswählen würde. Bevorzugt findet das Mischen mit Hilfe eines Rührers oder eines Mixers oder einer Kombination aus beidem statt. Bevorzugt wird das Mischen bei einer solchen Mischgeschwindigkeit durchgeführt, sodass eine homogene Durchmischung der Komponenten (V1) und (V2) zu der Polyurethanharnstoff-Mischung innerhalb von wenigen Minuten, bevorzugt innerhalb von weniger als 5 Minuten, oder bevorzugt innerhalb von weniger als 3 Minuten erfolgt.

Die Mischung mindestens der Komponenten (V1) und (V2) in Form des Klebstoffes weist bevorzugt eine ausreichend lange Verarbeitbarkeit auf, um die Mischung zu dem gewünschten Produkt in Form des Klebstoffs verarbeiten zu können. Eine ausreichend lange Verarbeitbarkeit ist mindestens dann noch gegeben, wenn die Mischung ausschließlich aufgrund der Schwerkraft aus einem Vorratsbehälter ausgießbar ist. Die Mischung kann bevorzugt innerhalb von 8 Stunden nach dem Mischen auf ein Substrat in Form der ersten weiteren Schicht aufgebracht werden. In dieser Zeit der ausreichend langen Verarbeitbarkeit kann die Komponente (V2) in Mischung mit (V1) nach Schritt (I) verweilen, ohne so weit abzureagieren, dass nach Herstellen des Filmes in Schritt (II) keine Vernetzung mehr stattfindet.

Das Aufbringen des bevorzugt amorphen Polyurethanharnstoffes auf die erste weitere Schicht in Schritt (II) kann mittels jeder Methode erfolgen, die der Fachmann hierfür auswählen würde. Bevorzugt findet das Aufbringen des bevorzugt amorphen Polyurethanharnstoffs in Form der wässrigen Polyurethanharnstoff-Dispersion durch eine Methode statt ausgewählt aus der Gruppe bestehend aus Drucken, Pinseln, Rakeln, Sprühen, Beschichten mit anderen bekannten Beschichtungsverfahren Die Polyurethanharnstoff-Dispersion kann in mehreren Lagen auf die erste weitere Schicht aufgebracht werden. Bevorzugt wird die Polyurethanharnstoff-Dispersion in 1 bis 10 Lagen, oder bevorzugt in 2 bis 10 Lagen, oder bevorzugt in 3 bis 10 Lagen auf die erste weitere Schicht aufgebracht. Die erste weitere Schicht ist beim Aufbringen der Polyurethanharnstoff-Dispersion bevorzugt so gelagert, dass sich die Polyurethanharnstoff-Dispersion möglichst gleichmäßig auf der Oberfläche der ersten weiteren Schicht verteilt. Nach Aufbringen der Polyurethanharnstoff-Dispersion auf die erste weitere Schicht wird der Vorläufer erhalten. Der Vorläufer kann bevor er dem zweiten Schritt (III) des Verfahrens zugeführt wird mit einer zweiten weiteren Schicht überdeckt werden. Das Überdecken kann jedoch auch nach dem Schritt (III) oder während des Schrittes (III) erfolgen.

Bevorzugt wird die Viskosität der Polyurethanharnstoff-Dispersion vor dem Aufbringen in Schritt (II) auf die benötigten Gegebenheiten durch Verdünnen oder Verdicken oder eine Kombination beider Methoden angepasst, um gewünschte Auftragsdicken zu erzielen. Dabei können als Zuschlagsstoffe L) bevorzugt in der Komponente (V2) Verdicker Verwendung finden. Typische Verdicker sind lösliche Polymere auf Polyacrylat- oder Polyurethanbasis, wie sie aus dem Stand der Technik bekannt sind. Bevorzugt sind Verdicker auf Basis von Polyurethanpolymeren. Zur Verdünnung der Polyurethanharnstoff-Dispersion können gängige Lösemittel, bevorzugt jedoch Wasser eingesetzt werden.

Typische weitere geeignete Zuschlags- und Hilfsstoffe L) sind Oberflächenadditive wie z.B. Benetzungshilfsmittel, Farbstoffe und/oder Verlaufshilfsmittel. Die Polyurethanharnstoff-Dispersion kann auch alle weiteren dem Fachmann für die jeweilige Anwendung bekannten Zuschlagstoffe enthalten.

Das Drucken kann jedes Druckverfahren der Polyurethanharnstoff-Dispersion beinhalten, das der Fachmann hierfür auswählen würde. Bevorzugt ist das Druckverfahren ausgewählt aus der Gruppe bestehend aus einem Siebdruckverfahren, einem Inkjet Verfahren, einem Tiefdruckverfahren, einem Offsetdruckverfahren, einem Walzendruckverfahren, einem Gravurdruckverfahren oder einer Kombination aus mindestens zwei hiervon. Beispiele für das Inkjet Druckverfahren sind das "Continuous Inkjet" Druckverfahren, bei dem das zu druckende Material in einem kontinuierlichen Strahl auf das Substrat aufgebracht wird oder das "Drop on Demand" Drucken, bei dem einzelne Tropfen auf das zu bedruckende Substrat aufgebracht werden. Mit all diesen Druckverfahren kann eine vollflächige Auftragung der Polyurethanharnstoff-Dispersion erfolgen oder auch eine teilflächige. Ebenso kann die Polyurethanharnstoff-Dispersion in einem bestimmten Muster aufgebracht werden, auch als "Pattern Coating" bezeichnet. Dabei kann die Dispersion durch alle dem Fachmann bekannten Verfahren, u.a. und insbesondere durch Gravurdruck, Siebdruck oder Inkjet-Druck aufgetragen werden.

Bevorzugt wird die Polyurethanharnstoff-Dispersion mit einem Auftragsgewicht in einem Bereich von 5 g/m² bis 200 g/m² auf das Substrat, hier die erste weitere Schicht oder die zweite weitere Schicht, aufgebracht.

Für den Rakelauftrag wird die erste weitere Schicht bevorzugt in einer Spannvorrichtung zuvor fixiert und anschließend kann das Rakel mit der Dispersion davorliegend per Hand oder automatisiert über die erste weitere Schicht geführt und dabei die Dispersion gleichmäßig auf diesem verteilt werden. Ebenso kann die Beschichtung über eine typische Rolle-zu-Rolle-Beschichtungsanlage mit Rakel erfolgen, bei der die erste weitere Schicht kontinuierlich beschichtet wird.

Beim Sprühverfahren wird die erste weitere Schicht bevorzugt in einen Rahmen gespannt und mit der Dispersion aus einer Sprühpistole ein- oder beidseitig besprüht. Der Auftrag kann in einem oder mehreren Kreuzgängen manuell oder über eine kontinuierliche Rolle-zu-Rolle-Sprühanlage erfolgen.

Die erste weitere Schicht ist bevorzugt so ausgestaltet wie für die erste weitere Schicht im Zusammenhang mit der erfindungsgemäßen Klebstoffschicht beschrieben.

Die thermische Behandlung in Schritt (III) kann auf jede Art und Weise erfolgen, wie sie der Fachmann hierfür auswählen würde. Bevorzugt findet die thermische Behandlung unter Anwendung von erhöhter Temperatur gegenüber Raumtemperatur statt. Die thermische Behandlung kann an jedem dafür geeigneten Ort stattfinden. Bevorzugt findet die thermische Behandlung in einem Raum ausgewählt aus der Gruppe bestehend aus einem Trockenraum, einem Trockenofen, einem Trockenrohr, oder einer Kombination aus mindestens zwei hiervon. Die thermische Trocknung kann durch IR- oder Mikrowellentrocknung ersetzt oder unterstützt werden. Die thermische Behandlung findet erfindungsgemäß bei einer Temperatur in einem Bereich von 20 °C bis 200 °C, bevorzugt in einem Bereich von 30 °C bis 200 °C, oder bevorzugt in einem Bereich von 50 °C bis 200 °C, oder bevorzugt in einem Bereich von 80 °C bis 200 °C, oder bevorzugt in einem Bereich von 20 °C bis 180 °C, oder bevorzugt in einem Bereich von 20 °C bis 150 °C, oder bevorzugt in einem Bereich von 20 °C bis 100 °C, oder bevorzugt in einem Bereich von 50 °C bis 150 °C statt. Bevorzugt findet die thermische Behandlung für einen Zeitraum in einem Bereich von 1 Minute bis 10 Stunden, oder bevorzugt in einem Bereich von 10 Minuten bis 5 Stunden, oder bevorzugt in einem Bereich von 30 Minuten bis 2 Stunden statt. Bevorzugt wird mindestens die Oberfläche der Polyurethanharnstoff-Dispersion mit einem Gas, bevorzugt Luft, überströmt, sodass eine schnellere Trocknung der Polyurethanharnstoff-Dispersion zu der Klebstoffschicht erfolgen kann.

Eine bevorzugte Ausgestaltung des zuvor beschriebenen Verfahrens, umfasst mindestens einen der folgenden weiteren Schritte:
(IV) Ablösen der Klebstoffschicht von der ersten weiteren Schicht;
(V) Übertragen der Klebstoffschicht von der ersten weiteren Schicht auf eine zweite weitere Schicht;
(VI) Überdecken der Klebstoffschicht mit einer zweiten weiteren Schicht auf der ersten Oberfläche der ersten Schicht;
(VII) Überdecken der Klebstoffschicht mit einer zweiten weiteren Schicht auf der ersten weiteren Oberfläche der ersten Schicht;
(VIII) Übertragen der Klebstoffschicht von der ersten weiteren Schicht auf ein Substrat;
(IX) Übertragen der Klebstoffschicht von der ersten weiteren Schicht auf mindestens einen Teil einer Bauteiloberfläche eines Bauteils;
(X) Übertragen der Klebstoffschicht von der ersten weiteren Schicht auf eine dritte weitere Schicht.

Das Ablösen der Klebstoffschicht in Schritt (IV) kann auf jede Art und Weise erfolgen, die der Fachmann hierfür vorsehen würde. Das Ablösen der Klebstoffschicht in Schritt (IV) erfolgt bevorzugt durch einen Spatel, beispielsweise einen Holz-, Plastik-, oder Metallspatel.

Das Übertragen der Klebstoffschicht von der ersten weiteren Schicht auf eine zweite weitere Schicht in Schritt (V) kann auf jede Art und Weise erfolgen, die der Fachmann hierfür vorsehen würde. Das Übertragen der Klebstoffschicht in Schritt (V) erfolgt bevorzugt durch Auflegen einer zweiten weiteren Schicht auf den nicht bedeckten Teil der Klebstoffschicht, wobei durch Druck auf die erste weitere Schicht die Klebstoffschicht übertragen wird. Der Druck kann beispielsweise durch Drehen der Klebstoffschicht erfolgen, sodass die zweite weitere Schicht die Klebstoffschicht trägt und allein durch die Schwerkraft auf dieser liegen bleibt, wenn die erste weitere Schicht entfernt wird. Ebenso kann Schritt (V) durch einen Umlaminierungsprozess, bevorzugt im kontinuierlichen Verfahren Rolle-zu-Rolle erfolgen. Der Laminierungsschritt erfolgt bevorzugt bei Temperaturen zwischen 5 °C und 200 °C.

Das Überdecken der Klebstoffschicht mit einer zweiten weiteren Schicht auf der ersten Oberfläche der ersten Schicht in Schritt (VI) kann auf jede Art und Weise erfolgen, die der Fachmann hierfür vorsehen würde. Das Überdecken der Klebstoffschicht in Schritt (VI) erfolgt bevorzugt durch Auflegen oder Auflaminieren einer zweiten weiteren Schicht auf der ersten Oberfläche der ersten Schicht.

Das Überdecken der Klebstoffschicht mit einer zweiten weiteren Schicht auf der ersten weiteren Oberfläche der ersten Schicht in Schritt (VII) kann auf jede Art und Weise erfolgen, die der Fachmann hierfür vorsehen würde. Das Überdecken der Klebstoffschicht in Schritt (VII) erfolgt bevorzugt durch Auflegen oder Auflaminieren einer zweiten weiteren Schicht auf der ersten weiteren Oberfläche der ersten Schicht.

Das Übertragen der Klebstoffschicht von der ersten weiteren Schicht auf ein Substrat in Schritt (VIII) kann auf jede Art und Weise erfolgen, die der Fachmann hierfür vorsehen würde. Das Übertragen der Klebstoffschicht in Schritt (VIII) erfolgt bevorzugt durch Auflegen des Substrats auf den nicht bedeckten Teil der Klebstoffschicht, wobei durch Druck auf die erste weitere Schicht die Klebstoffschicht auf das Substrat übertragen wird. Der Druck kann beispielsweise durch Drehen der Klebstoffschicht erfolgen, sodass das Substrat die Klebstoffschicht trägt und allein durch die Schwerkraft auf dieser liegen bleibt, wenn die erste weitere Schicht entfernt wird. Bevorzugt wird ein zusätzlicher Druck mit Hilfe eines Gegenstandes auf mindestens einem Teil der ersten weiteren Schicht erzeugt, der eine bessere Haftung der Klebstoffschicht auf dem Substrat als auf der ersten weiteren Schicht bewirkt. Bevorzugt erfolgt Schritt (VIII) durch einen Umlaminierungsprozess, bevorzugt im kontinuierlichen Verfahren Rolle-zu-Rolle.

Das Übertragen der Klebstoffschicht von der ersten weiteren Schicht auf mindestens einen Teil einer Bauteiloberfläche eines Bauteils in Schritt (IX) kann auf jede Art und Weise erfolgen, die der Fachmann hierfür vorsehen würde. Das Übertragen der Klebstoffschicht in Schritt (IX) erfolgt bevorzugt durch Auflegen der Bauteiloberfläche des Bauteils auf den nicht bedeckten Teil der Klebstoffschicht, wobei durch Druck auf die erste weitere Schicht die Klebstoffschicht auf das Bauteil übertragen wird. Der Druck kann beispielsweise durch Drehen der Klebstoffschicht erfolgen, sodass das Bauteil die Klebstoffschicht trägt und allein durch die Schwerkraft auf dieser liegen bleibt, wenn die erste weitere Schicht entfernt wird. Bevorzugt wird ein zusätzlicher Druck mit Hilfe eines Gegenstandes auf mindestens einem Teil der ersten weiteren Schicht erzeugt, der eine bessere Haftung der Klebstoffschicht auf dem Substrat als auf der ersten weiteren Schicht bewirkt. Bevorzugt erfolgt Schritt (IX) durch einen Umlaminierungsprozess, bevorzugt im kontinuierlichen Verfahren Rolle-zu-Rolle.

Das Übertragen der Klebstoffschicht von der ersten weiteren Schicht auf eine dritte weitere Schicht in Schritt (X) kann auf jede Art und Weise erfolgen, die der Fachmann hierfür vorsehen würde. Das Übertragen der Klebstoffschicht in Schritt (X) erfolgt bevorzugt durch Auflegen der dritten weiteren Schicht auf den nicht bedeckten Teil der Klebstoffschicht, wobei durch Druck auf die erste weitere Schicht die Klebstoffschicht übertragen wird. Der Druck kann beispielsweise durch Drehen der Klebstoffschicht erfolgen, sodass die dritte weitere Schicht die Klebstoffschicht trägt und allein durch die Schwerkraft auf dieser liegen bleibt, wenn die erste weitere Schicht entfernt wird. Ebenso kann Schritt (X) durch einen Umlaminierungsprozess, bevorzugt im kontinuierlichen Verfahren Rolle-zu-Rolle erfolgen. Der Laminierungsschritt erfolgt bevorzugt bei Temperaturen zwischen 5 °C und 200 °C.

Bevorzugt sind das Material, die Eigenschaften und die Form der dritten weiteren Schicht so ausgestaltet, wie für die erste weitere Schicht zuvor beschrieben. Die dritte weitere Schicht ist bevorzugt gleich der ersten oder zweiten weiteren Schicht aufgebaut.

Ein weiterer Gegenstand der Erfindung betrifft die Verwendung des erfindungsgemäßen Klebstoffs oder der erfindungsgemäßen Klebstoffschicht zur Befestigung eines Produktes auf einem Gegenstand oder der Haut eines Lebewesens.

Bei der Verwendung des erfindungsgemäßen Klebstoffes wird bevorzugt mindestens einer der folgenden Schritte durchgeführt:
❖ Der Klebstoff wird mit dem Produkt in Kontakt gebracht. Bevorzugt haftet der Klebstoff beim Inkontaktbringen mit dem Produkt mehr an dem Produkt als an der ersten weiteren Schicht, der zweiten weiteren Schicht oder der dritten weiteren Schicht, auf dem der Klebstoff in Form der Klebstoffschicht produziert oder auf den der Klebstoff übertragen wurde.
❖ Das Produkt wird mit einem weiteren Gegenstand, einem weiteren Produktteil oder der Haut eines Verwenders in Kontakt gebracht. Das Produkt haftet bevorzugt nach dem Inkontaktbringen mit dem Gegenstand, dem weiteren Produktteil oder der Haut so stark mit deren Oberfläche, dass es bei gewöhnlicher Benutzung des Produktes sich nicht wieder hiervon löst. Unter gewöhnlicher Benutzung wird die marktübliche Verwendung des Produktes verstanden, wie der Fachmann sie verstehen würde. Hierzu zählen alle alltäglichen Verrichtungen wie Duschen, normale Bewegung, normale Tätigkeiten des Patienten.
❖ Das Produkt wird nach seiner gewünschten Tragezeit unter Aufwand einer Kraft in einem Bereich von 0,1 bis 10,0 N/20 mm, bevorzugt 0,15 bis 5 N/20 mm von dem Substrat entfernt. Das Substrat kann auch die Haut des Verwenders sein.

Bevorzugt handelt es sich bei dem zu befestigenden Produkt um eines der zuvor beschriebenen Produkte.

Bevorzugt findet die Verwendung des erfindungsgemäßen Klebstoffs in und auf einem Produkt im medizinischen Bereich, insbesondere zur Befestigung des Produktes auf der Haut eines Lebewesens statt. Insbesondere im Bereich der medizinischen, besonders der chirurgischen Anwendungen, ist es wünschenswert eine gute Klebkraft der medizinischen Produkte auf der Haut des Lebewesens, insbesondere des menschlichen oder tierischen Patienten, zu gewährleisten. Bevorzugt weist das medizinische Produkt weiterhin folgende Merkmale auf:
(1) eine Verweildauer auf der Haut in einem Bereich von 1 Sekunde bis 180 Tage:
(2) eine Minderung der Klebkraft über die Verweildauer von 10 Tagen von weniger als 50 %, bevorzugt von weniger als 30 %, oder bevorzugt von weniger als 10 %, bezogen auf die ursprüngliche Klebkraft.

Aus dem erfindungsgemäßen Klebstoff wird bevorzugt eine Klebstoffschicht gebildet, umfassend
- mindestens eine erste Schicht, umfassend mindestens eine erste Oberfläche und mindestens eine erste weitere Oberfläche, wobei die erste Oberfläche im Wesentlichen parallel zur ersten weiteren Oberfläche verläuft,
wobei die erste Schicht den zuvor beschriebenen erfindungsgemäßen Klebstoff beinhaltet.

Bevorzugt beinhaltet die Klebstoffschicht den Klebstoff in einem Bereich von 60 bis 100 Gew.-%, oder bevorzugt in einem Bereich von 70 bis 100 Gew.-%, oder bevorzugt in einem Bereich von 80 bis 100 Gew.-%, bezogen auf das Gesamtgewicht der Klebstoffschicht.

Die Klebstoffschicht beinhaltet bevorzugt mindestens eine weitere Komponente in einem Bereich von 0 bis 40 Gew.-%, oder bevorzugt in einem Bereich von 0 bis 30 Gew.-%, oder bevorzugt in einem Bereich von 0 bis 20 Gew.-%, bezogen auf das Gesamtgewicht der Klebstoffschicht. Weiterhin bevorzugt beinhaltet die Klebstoffschicht die weitere Komponente in einem Bereich von 0,1 bis 20 Gew.-%, oder bevorzugt in einem Bereich von 0,5 bis 15 Gew.-%, oder bevorzugt in einem Bereich von 1 bis 10 Gew.-%, bezogen auf die Gesamtmasse der Klebstoffschicht. Die weitere Komponente kann ausgewählt sein aus der Gruppe bestehend aus Wasser, einem Verdicker, einem Verdünner, einem Füller, wie Calcit oder Talkum), einem Farbstoff, einem Superabsorber (beispielsweise basierend auf einem Polyacrylat oder Carboxymethylcellulose), einem antimikrobiellen oder pharmazeutisch wirksamen Stoff (beispielsweise growth factors, Peptide, Schmerzmittel, Mittel, die die Wundheilung beschleunigen, Silber, Polyhexanid u.a.), einem Desinfektionsmittel, wie einem Bakterizid oder Fungizid, oder einer Kombination aus mindestens zwei davon.

Die Klebstoffschicht weist bevorzugt eine Dicke in einem Bereich von 2 µm bis 20 mm, oder bevorzugt in einem Bereich von 5 µm bis 10 mm, oder bevorzugt in einem Bereich von 10 µm bis 2000 µm, oder bevorzugt in einem Bereich von 40 µm bis 800 µm. Bevorzugt weist die Klebstoffschicht über ihre gesamte, bevorzugt flächenförmige, Ausdehnung die gleiche Dicke auf. Unter gleicher Dicke wird erfindungsgemäß verstanden, dass die Dicke über die gesamte Klebstoffschicht nicht mehr als 10 % von der mittleren Dicke der Klebstoffschicht abweicht. Die mittlere Dicke der Klebstoffschicht ist gemäß der Erfindung das Mittel aus den ermittelten Werten der Dicke an der jeweils dünnsten und der jeweils dicksten Stelle der Klebstoffschicht. Die Werte für die Dickenbestimmung sind mittels einer handelsüblichen Mikrometerschraube bestimmbar.

Die Klebstoffschicht weist in ihrer größten Flächenausdehnung bevorzugt eine Form auf, ausgewählt aus der Gruppe bestehend aus rund, eckig, rechtwinklig, quadratisch, elliptisch, trapezförmig, rautenförmig oder einer Kombination aus mindestens zwei hiervon. Bevorzugt ist die Klebstoffschicht rechteckig oder elliptisch.

Die Klebstoffschicht weist bevorzugt eine Breite in einem Bereich von 1 mm bis 10 m, oder bevorzugt in einem Bereich von 1 cm bis 10 m, oder bevorzugt in einem Bereich von 1 m bis 10 m, oder bevorzugt in einem Bereich von 1 mm bis 5 m, oder bevorzugt in einem Bereich von 1 mm bis 1 m, oder bevorzugt in einem Bereich von 5 mm bis 5 m, oder bevorzugt in einem Bereich von 1 cm bis 1 m auf.

Die Klebstoffschicht weist bevorzugt eine Länge in einem Bereich von 2 mm bis 100 m, oder bevorzugt in einem Bereich von 1 cm bis 10 m, oder bevorzugt in einem Bereich von 10 cm bis 10 m, oder bevorzugt in einem Bereich von 2 mm bis 50 m, oder bevorzugt in einem Bereich von 2 mm bis 10 m, oder bevorzugt in einem Bereich von 5 mm bis 50 m, oder bevorzugt in einem Bereich von 1 cm bis 10 m auf.

Die Klebstoffschicht weist erfindungsgemäß mindestens zwei Oberflächen auf, eine erste Oberfläche sowie eine erste weitere Oberfläche. Die erste Oberfläche und die erste weitere Oberfläche verlaufen erfindungsgemäß im Wesentlichen parallel zueinander. Im Rahmen der Erfindung wird unter im Wesentlichen parallel verstanden, dass sich die beiden Oberflächen an keiner Stelle des Verlaufs der der Klebstoffschicht berühren. Bevorzugt werden die beiden im Wesentlichen parallel zueinander verlaufenden Oberfläche, nämlich die erste Oberfläche und die erste weitere Oberfläche an den Rändern der Klebstoffschicht durch mindestens eine zweite weitere Oberfläche voneinander getrennt. Die Ausdehnung dieser zweiten weiteren Oberfläche im Lot zwischen der ersten Oberfläche und der ersten weiteren Oberfläche bildet an den Rändern der Klebstoffschicht deren Dicke. Bevorzugt weisen die erste Oberfläche sowie die erste weitere Oberfläche eine flächenförmige Ausdehnung auf. Weiterhin bevorzugt weisen die erste Oberfläche und die erste weitere Oberfläche eine nahezu identische Gesamtfläche auf. Unter nahezu identischer Gesamtfläche wird eine Abweichung der Gesamtflächen der ersten zu der ersten weiteren Oberfläche von nicht mehr als 50 %, bevorzugt nicht mehr als 30 %, oder bevorzugt nicht mehr als 10 %, oder bevorzugt nicht mehr als 5 %, bezogen auf die Gesamtfläche der ersten Oberfläche verstanden.

Die Form der Oberfläche der ersten Oberfläche und/oder der ersten weiteren Oberfläche ist bevorzugt ausgewählt aus der Gruppe bestehend aus plan, gebogen, geknickt oder einer Kombination aus mindestens zwei hiervon. Die erste Oberfläche und/oder die erste weitere Oberfläche können sich jeweils auch über mehrere, bevorzugt zwei, drei oder vier aneinander grenzenden Flächen erstrecken. Bevorzugt weisen die aneinander grenzenden Flächen, die die Oberfläche der ersten Oberfläche und/oder ersten weiteren Oberfläche eine Neigung zueinander in einem Bereich von weniger als 45°, oder bevorzugt von weniger als 40°, oder bevorzugt von weniger als 30° zueinander auf. Die Oberfläche der ersten Oberfläche und/oder der ersten weiteren Oberfläche ist bevorzugt plan.

Die Gesamtfläche der ersten Oberfläche und/oder der ersten weiteren Oberfläche liegt bevorzugt in einem Bereich von 1 mm² bis 1000 m², oder bevorzugt in einem Bereich von 100 mm² bis 500 m², oder bevorzugt in einem Bereich 1 cm² bis 100 m².

Die Klebstoffschicht weist bevorzugt mindestens eines der folgenden Merkmale auf:
a) eine Wasserdampfdurchlässigkeit in einem Bereich von mindestens 800 g/d m², bevorzugt ≥ 1200 g/d m² und besonders bevorzugt ≥ 1500 g/d m².
b) eine Länge und eine Breite die mindestens das 10-fache, bevorzugt mindestens das 20-, oder bevorzugt mindestens das 30-, oder bevorzugt mindestens das 40-fache, oder bevorzugt mindestens das 100-fache der Dicke der Klebstoffschicht entspricht;
c) eine Klebekraft in einem Bereich von ≥ 0,25 N/20 mm, oder bevorzugt von ≥ 0,5 N/20 mm, oder bevorzugt von ≥ 1,0 N/20 mm, oder bevorzugt von ≥ 1,3 N/20 mm (ermittelt über 90° Peel Test gegen Alublech, DIN EN 1464);

Bevorzugt liegen die so ermittelten Klebkräfte in einem Bereich von 0,25 bis 20 N/20 mm, oder bevorzugt von 0,5 bis 15 N/20 mm, oder bevorzugt von 1,0 bis 12,5 N/20 mm, oder bevorzugt von 1,3 bis 12,0 N/20 mm, oder bevorzugt von 1,5 bis 10,0 N/20 mm.

In einer bevorzugten Ausgestaltung der Klebstoffschicht, ist die Klebstoffschicht auf ihrer ersten Oberfläche mindestens von einer ersten weiteren Schicht mindestens zu einem Teil überlagert.

Bevorzugt überlagert die erste weitere Schicht die erste Oberfläche der Klebstoffschicht in einem Bereich von 50 bis 100 %, oder bevorzugt in einem Bereich von 60 bis 100 %, oder bevorzugt in einem Bereich von 70 bis 100 %, oder bevorzugt in einem Bereich von 80 bis 100 %, bezogen auf die Gesamtfläche der ersten Oberfläche der Klebstoffschicht. Bevorzugt weist die erste weitere Schicht mindestens eine erste Schichtoberfläche sowie eine weitere Schichtoberfläche auf. Die erste und/oder weitere Schichtoberfläche der ersten weiteren Schicht weist bevorzugt eine Fläche in einem Bereich von 1 mm² bis 1000 m², oder bevorzugt in einem Bereich von 100 mm² bis 500 m², oder bevorzugt in einem Bereich 1 cm² bis 100 m². Bevorzugt weist die erste und/oder weitere Schichtoberfläche der ersten weiteren Schicht eine Gesamtoberfläche auf, die größer ist als die Gesamtoberfläche der ersten Oberfläche der Klebstoffschicht. Bevorzugt weist die erste und/oder weitere Schichtoberfläche der ersten weiteren Schicht eine Gesamtoberfläche in einem Bereich von 105 bis 200 %, oder bevorzugt in einem Bereich von 110 bis 190 %, oder bevorzugt in einem Bereich von 120 bis 180 %, bezogen auf die Gesamtoberfläche der ersten Oberfläche der Klebstoffschicht, auf. In einer weiteren bevorzugten Ausführung weist die erste und/oder weitere Schichtoberfläche der ersten weiteren Schicht eine Gesamtoberfläche auf, die gleich groß ist wie die Gesamtoberfläche der ersten Oberfläche der Klebstoffschicht.

Bevorzugt steht die erste Schichtoberfläche der ersten weiteren Schicht mit der ersten Oberfläche der Klebstoffschicht in direktem Kontakt. Alternativ kann mindestens zu einem Teil zwischen der ersten weiteren Schicht und der Klebstoffschicht ein zusätzliches Material, wie ein Primer, beispielsweise auf Alkyd- oder Acrylharz Basis, angeordnet sein. Die erste weitere Schicht wird bevorzugt vor Kontaktierung durch die Klebstoffschicht mittels einer Oberflächenbehandlungsmethode ausgewählt aus der Gruppe bestehend aus Plasmabehandlung, Ozonbehandlung und Coronabehandlung oder einer Kombination aus mindestens zwei hiervon, vorbehandelt.

Die mindestens eine erste weitere Schicht beinhaltet bevorzugt ein Material ausgewählt aus der Gruppe bestehend aus einem Polymer, einem Vliesstoff (auch non-woven genannt), einem Gewebe, einem Glas, einem Metall, einer Keramik, einem Mineral, einem Papier oder einer Kombination oder Mischung von mindestens zwei hieraus.

Das Polymer kann jedes Polymer sein, das der Fachmann für die erste weitere Schicht auswählen würde. Das Polymer ist bevorzugt ausgewählt aus der Gruppe bestehend aus einem Polyvinylchlorid, einem Polyolefin, wie Polyethylen oder Polypropylen, einem Polyimid, einem Polyethylenterephthalat, einem Polybutylenterephthalat, einem Polycarbonat, einem Polyamid, einem Polyurethan, wie einem thermoplastischen Polyurethan, einem Silikon oder einer Mischung oder Kombination aus mindestens zwei hiervon. Besonders bevorzugt ist das Polymer ausgewählt aus der Gruppe bestehend aus einem Polyester, einem Polyolefine, einem Polyvinylchlorid, einem Silikon, einem thermoplastischen Polyurethan, darunter besonders bevorzugt ein thermoplastisches Polyurethan.

Der Vliesstoff kann jeder Vliesstoff sein, den der Fachmann für die erste weitere Schicht auswählen würde. Der Vliesstoff ist bevorzugt ausgewählt aus der Gruppe bestehend aus pflanzlichen Fasern, wie Baumwolle, tierischen Fasern, wie Wolle, Chemiefasern aus natürlichen Polymeren, wie Viskose, Chemiefasern aus synthetischen Polymeren, wie Polyestervliesstoffe, und Chemiefasern aus mineralischen Stoffen, wie Glasfaservliesstoff, Carbonfaservliesstoff, Edelstahlfaservliesstoff, Basaltfaservliesstoff oder einer Mischung von mindestens zwei hiervon.

Das Gewebe kann jedes Gewebe sein, das der Fachmann für die erste weitere Schicht auswählen würde. Das Gewebe ist bevorzugt ausgewählt aus der Gruppe bestehend aus einem Baumwollgewebe, einem Wollgewebe oder einer Kombination aus mindestens zwei hieraus.

Das Glas kann jedes Glas sein, das der Fachmann für die erste weitere Schicht auswählen würde. Das Glas kann ein metallisches Glas oder ein nicht-metallisches Glas sein. Bevorzugt ist das Glas ein nicht-metallisches Glas. Das Glas ist bevorzugt ausgewählt aus der Gruppe bestehend aus einem silikatischen Glas, wie Quarzglas, einem Boratglas, einem Phosphatischen Glas, einem Chalkogenitglas, einem Halogenidglas oder einer Mischung aus mindestens zwei hieraus.

Das Metall kann jedes Metall sein, das der Fachmann für die erste weitere Schicht auswählen würde. Das Metall ist bevorzugt ausgewählt aus der Gruppe bestehend aus Kupfer, Eisen, Silber, Gold, Platin, Palladium, Nickel, einer Bronze-Legierung, einer Messing-Legierung oder einer Mischung oder Kombination von mindestens zwei hieraus.

Die Keramik kann jede Keramik sein, die der Fachmann für die erste weitere Schicht auswählen würde. Die Keramik ist bevorzugt eine Oxid-Keramik oder eine Nicht-Oxid-Keramik. Die Oxid-Keramik ist bevorzugt ausgewählt aus der Gruppe bestehend einer Aluminiumoxidkeramik, wie Korund, einer Berylliumoxidkeramik, einer Zirconium(IV)-oxid-Keramik, einer Titan(IV)-oxid-Keramik, einer Aluminiumtitan-Keramik, einer Bariumtitanat-Keramik oder einer Mischung oder Kombination aus mindestens zwei hieraus. Die Nicht-Oxid-Keramik ist bevorzugt ausgewählt aus der Gruppe bestehend aus einem Siliciumcarbin, einem Bornitrid, einem Borcarbid, einem Siliciumnitrid, einem Aluminiumbitrid, einem Molybdänsilicid, einem Wolframcarbin oder einer Mischung oder Kombination aus mindeste zwei hiervon.

Das Mineral kann jedes Mineral sein, das der Fachmann für die erste weitere Schicht auswählen würde. Unter einem Mineral wird gemäß der Erfindung ein Element oder eine chemische Verbindung verstanden, die im Allgemeinen kristallin und durch geologische Prozesse gebildet worden ist. Der Begriff "chemische Verbindung" beinhalten eine feste Zusammensetzung und eine definierte chemische Struktur. Stoffgemische sind keine Minerale. Die Zusammensetzungen von Mineralen können jedoch eine gewisse Variation aufweisen (Mischkristalle), solange sie strukturell homogen sind.

Das Mineral kann aus organischen Bestandteilen aufgebaut sein oder aus anorganischen Bestandteilen oder aus einer Kombination aus beiden. Die organischen Bestandteile sind bevorzugt ausgewählt aus der Gruppe bestehend aus Mellit, Evenkit, Whewellit, Weddellit oder eine Mischung aus mindestens zwei hiervon. Die anorganischen Bestandteile sind bevorzugt ausgewählt aus der Gruppe bestehend aus Borax, Bernstein, Kalifeldspat, Feldspat, Clacit, Kaolinit oder einer Kombination aus mindestens zwei hiervon.

Das Papier kann jedes Papier sein, das der Fachmann für die erste weitere Schicht auswählen würde. Das Papier ist bevorzugt ausgewählt aus der Gruppe bestehend einem Naturpapier und einem synthetischen Papier oder einer Kombination hieraus. Das Naturpapier beinhaltet als einen Hauptbestandteil Cellulose. Das synthetische Papier kann darüber hinaus ein Polymer beinhalten. Das Polymer ist bevorzugt ausgewählt aus der Gruppe der Polymere, wie oben beschrieben. Das Papier weist bevorzugt eine flächenbezogene Masse in einem Bereich von 20 bis 500 g/m², oder bevorzugt in einem Bereich von 20 bis 400 g/m², oder bevorzugt in einem Bereich von 20 bis 200 g/m², oder bevorzugt in einem Bereich von 50 bis 500 g/m², oder bevorzugt in einem Bereich von 100 bis 500 g/m², oder bevorzugt in einem Bereich von 200 bis 500 g/m² auf.

Das Papier weist bevorzugt eine Beschichtung auf. Die Beschichtung beinhaltet bevorzugt ein Polymer ausgewählt aus der Gruppe, wie bereits oben beschrieben. Bevorzugt überlagert das Polymer das Papier zu mindestens 50 %, oder bevorzugt zu mindestens 60 %, oder zu mindestens 80 %, bezogen auf die Gesamtoberfläche des Papiers. Besonders bevorzugt überlagert das Polymer das Papier auf dessen Gesamtoberfläche. Bevorzugt ist das Polymer ein Silikon oder ein Polyolefin, das ein Wachs auf dem Papier ausbildet. Die Parameter zur Beschichtung des Papiers durch einen Silikon- oder Polyolefinwachs wird bevorzugt so gewählt, dass die Klebstoffschicht rückstandsfrei von dem Papier ablösbar ist.

Die Oberflächenrauheit der ersten weiteren Schicht, insbesondere der Papierschicht, liegt bevorzugt in einem Bereich von Rz < 2000 nm, bevorzugt < 1500 nm, oder bevorzugt < 1000 nm. Die Oberflächenrauheit wird mittels Weißlichtinterferometrie (Messung im PSI-Modus) in Anlehnung an DIN EN ISO 25178, Part 6 ermittelt.

In einer bevorzugten Ausgestaltung der Klebstoffschicht ist die Klebstoffschicht auf ihrer ersten weiteren Oberfläche mindestens von einer zweiten weiteren Schicht mindestens zu einem Teil überlagert. Die zweite weitere Schicht kann aus jedem Material gefertigt sein, das der Fachmann für die zweite weitere Schicht auswählen würde. Die zweite weitere Schicht weist bevorzugt die gleichen Bestandteile, Materialien, Eigenschaften, Formen und Dimensionen auf wie für die erste weitere Schicht beschrieben.

Bevorzugt stehen die erste weitere Schicht und/oder die zweite weitere Schicht in direktem Kontakt zu der ersten Schicht. Alternativ kann zwischen der ersten weiteren Schicht und/oder der zweiten weiteren Schicht und der ersten Schicht eine dritte weitere Schicht angeordnet sein. Die Eigenschaften, Materialien, Formen und Dimensionen der dritten weiteren Schicht sind bevorzugt aus der Liste ausgewählt, wie für die erste weitere Schicht beschrieben.

Bevorzugt sind die erste weitere Schicht und/oder die zweite weitere Schicht leicht von der Klebstoffschicht ablösbar. Unter leicht ablösbar wird erfindungsgemäß verstanden, dass ein Verwender der Klebstoffschicht das Ablösen der ersten weiteren Schicht und/oder der zweiten weiteren Schicht ohne merkbaren Kraftaufwand vornehmen kann. Bevorzugt liegt der Kraftaufwand zum Ablösen der ersten weiteren Schicht und/oder zweiten weiteren Schicht in einem Bereich von 0,02 bis 2 N/ 10 mm, bevorzugt bei 0,05 bis 1 N/ 10 mm.

Die erste weitere und/oder die zweite weitere Schicht sind bevorzugt so ausgestaltet, dass sie die Klebstoffschicht vor äußeren Einflüssen, wie Staub, Flüssigkeiten, Feuchtigkeit, Temperatur, Druck und anderen Einflüssen schützen. Die erste weitere und/oder die zweite weitere Schicht werden bevorzugt für Transportzwecke der Klebstoffschicht über die Klebstoffschicht überlagert. Die erste weitere und/oder die zweite weitere Schicht dienen vor allem dem leichten Übertragen der Klebstoffschicht auf die Oberfläche eines Produktes.

Bevorzugt ist ein Produkt, wobei das Produkt einen Klebstoff, wie er zuvor beschrieben wurde, beinhaltet und weiterhin mindestens eines der folgenden Merkmale aufweist:
- mindestens ein Substrat,
- mindestens ein Bauteil, beinhaltend mindestens eine Bauteiloberfläche.

Weiterhin ist es bevorzugt, dass das Produkt mindestens eines, bevorzugt zwei, oder bevorzugt alle der folgenden Merkmale aufweist:
- mindestens eine erste Schicht,
- mindestens eine erste weitere Schicht,
- mindestens eine zweite weitere Schicht.

Das Produkt kann jedes Produkt sein, das der Fachmann auswählen würde, welches eine Klebstoffschicht aufweisen kann. Bevorzugt ist das Produkt ausgewählt aus der Gruppe bestehend aus einem medizinischen Produkt, einem Haushaltsprodukt, einem Verkehrsmittel, einem Kommunikationsmittel, einem oder einer Kombination aus mindestens zwei hiervon.

Das medizinische Produkt kann jedes Produkt sein, das der Fachmann für medizinische Zwecke einsetzen würde. Unter medizinischem Produkt wird erfindungsgemäß jedes Produkt verstanden, das durch medizinisches Personal, wie Ärzte, Krankenschwestern, Arzthelferinnen usw. am Patienten angewendet wird oder der Patient selber zur Überwachung eines Parameters, zur Behandlung einer Krankheit oder Wunde oder zur Verbesserung seines Gesundheitszustandes an sich oder einer anderen Person anwendet. Das medizinische Produkt ist bevorzugt ausgewählt aus der Gruppe bestehend aus einem medizinischen Gerät, einem medizinischen Artikel oder einer Kombination hieraus. Im Unterschied zum medizinischen Artikel weist das medizinische Gerät eine Stromversorgung oder zumindest eine Einrichtung zur Verbindung mit einer Stromversorgung auf.

Das medizinische Gerät kann jedes Gerät sein, das der Fachmann zur Untersuchung oder Behandlung eines Patienten auswählen würde. Das medizinische Gerät ist bevorzugt ausgewählt aus der Gruppe bestehend aus einem diagnostischen Gerät, einem therapeutischen Gerät, einem chirurgischen Gerät oder einer Kombination aus mindestens zwei hiervon. Beispiele für diagnostische Geräte sind ein Temperaturmessgerät, ein Blutdruckmessgerät, ein Pulsmessgerät, ein Blutzuckermessgerät sowie deren Befestigungselemente am Körper des Nutzers. Beispiele für therapeutische Geräte sind ein Gerät zur Unterdruckwundbehandlung, einem Herzschrittmacher, einer Insulinpumpe, einem Defibrillator, z.B. einem implantierbaren Defibrillator, oder eine Kombination aus mindestens zwei hiervon. Beispiele für ein chirurgisches Gerät sind Zahnbehandlungsgeräte wie Zahnbohrer, elektrisches Skalpell oder Kombination aus mindestens zwei hiervon.

Der medizinische Artikel kann jeder Artikel sein, den der Fachmann zu Behandlung eines Patienten auswählen würde. Der medizinische Artikel ist bevorzugt ausgewählt aus der Gruppe bestehend aus einem Katheter, einem Behältnis für einen künstlichen Darmausgang, einem medizinischen Band (tape), einem Skalpell, einer Spritze, einer Kanüle, einem Wundbehandlungsmittel, wie einem Pflaster, einer medizinischen Binde, einem wiederverwertbarem Stofftuch, einem Einmal-Stofftuch oder einer Kombination aus mindestens zwei hiervon. Die charakteristische Eigenschaft der medizinischen Binde, dem wiederverwertbaren Stofftuch, dem Einmal-Stofftuch ist ihre Fähigkeit zur Aufnahme von Flüssigkeiten, insbesondere Blut, wie sie bei diagnostischen, therapeutischen oder chirurgischen Eingriffen stattfinden können. Die Unterscheidung zwischen der Binde und dem Stofftuch erfolgt aufgrund ihrer Materialzusammensetzung. Die Binde kann beispielsweise sowohl natürliche Materialien, wie Baumwolle und/oder Wolle in Kombination mit künstlichen Materialien enthalten, während das Stofftuch rein aus Baumwolle besteht.

Bevorzugt ist das Produkt ausgewählt aus der Gruppe bestehend aus einem Behältnis für einen künstlichen Darmausgang, einem Wundbehandlungsmittel, einem medizinischen Band (tape), einem Gerät zur Unterdruckwundbehandlung und einem wearable device, also einem tragbaren, elektronischen, medizinischen Gerät, z.B. ein Blutdruckmesser oder ein anderer Sensor, der zur Überwachung des Nutzers auf dessen Haut geklebt wird, oder einer Kombination aus mindestens zwei hieraus.

Das Haushaltsprodukt kann jedes Haushaltsprodukt sein, das der Fachmann für diesen Zweck auswählen würde. Das Haushaltsprodukt ist bevorzugt ausgewählt aus der Gruppe bestehend aus einem Mixer, einem Rührgerät, einer Schneidemaschine, einer Anrichteplatte oder einer Kombination aus mindestens zwei hiervon.

Das Verkehrsmittel kann jedes Verkehrsmittel sein, das der Fachmann für diesen Zweck auswählen würde. Das Verkehrsmittel ist bevorzugt ausgewählt aus der Gruppe bestehend aus einem Auto, einem Flugzeug, einem Motorrad, einem Fahrrad, einem Roller, einem Inline-Skater oder einer Kombination aus mindestens zwei hiervon.

Das Kommunikationsmittel kann jedes Kommunikationsmittel sein, das der Fachmann für diesen Zweck auswählen würde, insbesondere einem Gerät, das zur Datenübermittlung dient. Das Kommunikationsmittel ist bevorzugt ausgewählt aus der Gruppe bestehend aus einem Telefon, einem mobilen Telefon, einem Faxgerät, einem Modem, einem Computer, einem GPS-Gerät, einem Navigationsgerät oder einer Kombination aus mindestens zwei hiervon.

Das Produkt kann den Klebstoff für verschiedene Zwecke beinhalten. Das Produkt beinhaltet den Klebstoff bevorzugt zur Verbindung von Produktteilen untereinander. In einer zusätzlichen oder alternativen Ausgestaltung des Produkts, beinhaltet das Produkt den Klebstoff, um das Produkt an ein weiteres Produkt, einen weiteren Gegenstand oder an die Haut eines Nutzers des Produktes zu befestigen. Der Gegenstand kann jeder Gegenstand sein, den der Fachmann mit dem Produkt verbinden würde. Bevorzugt dient der Klebstoff, den das Produkt beinhaltet, um das Produkt an der Haut eines Nutzers zu befestigen.

Das Substrat kann jedes Substrat sein, das der Fachmann für ein erfindungsgemäßes Produkt auswählen würde. Das Substrat beinhaltet bevorzugt ein Material ausgewählt aus der Gruppe bestehend aus einem Polymer, einem Metall, einem Gewebe, einem Vlies, einem Mineral oder einer Kombination aus mindestens zwei hiervon. Das Material des Substrats, wie das Polymer, das Metall, das Gewebe, das Vlies, das Mineral oder deren Kombinationen sind bevorzugt aus der Gruppe der Materialien ausgewählt, wie bereits für die erste weitere Schicht beschrieben. Das Substrat kann jede Form aufweisen, die der Fachmann für das Substrat auswählen würde. Bevorzugt weist das Substrat eine flächige Form auf. Bevorzugt weist das Substrat eine Dicke in einem Bereich von 10 µm bis 10 cm, oder bevorzugt in einem Bereich von 100 µm bis 10 cm, oder bevorzugt in einem Bereich von 1 mm bis 10 cm, oder bevorzugt in einem Bereich von 10 µm bis 1 cm, oder bevorzugt in einem Bereich von 10 µm bis 1 mm, oder bevorzugt in einem Bereich von 1 mm bis 1 cm. Das Substrat weist bevorzugt eine flexible Struktur auf. Bevorzugt ist das Substrat so flexibel, dass es sich den Konturen eines Körpers eines Menschen anpassen kann.

Bevorzugt ist das das Substrat ausgewählt aus der Gruppe bestehend aus einer Folie, v.a. eine thermoplastische PU-Folie, einem Trennpapier, einem Vlies (Non-Woven z.B. für Klebebänder), einem PU-Schaum oder einer Kombination aus mindestens zwei hiervon

Das Bauteil kann jedes Bauteil sein, das der Fachmann für ein erfindungsgemäßes Produkt auswählen würde. Das Bauteil ist bevorzugt ausgewählt aus der Gruppe bestehend aus einem Gerätebauteil der Geräte, die zuvor im Zusammenhang mit dem Produkt beschrieben wurden. Das Material aus dem das Bauteil, insbesondere die mindestens eine Bauteiloberfläche, besteht ist bevorzugt aus der Gruppe der Materialien ausgewählt, wie bereits für die erste weitere Schicht beschrieben.

Das Bauteil kann einen Teil des Produktes darstellen oder das gesamte Produkt. Der Klebstoff dient bevorzugt dazu verschiedene Bauteile des Produktes miteinander zu dem Produkt zu verbinden. Alternativ oder zusätzlich kann der Klebstoff dazu dienen das Bauteil mit der Haut eines Nutzers des Produktes zu verbinden. Wiederum alternativ oder zusätzlich kann der Klebstoff dazu dienen, das Produkt mit einem weiteren Gegenstand zu verbinden. Der Gegenstand kann jeder Gegenstand sein, den der Fachmann mit dem Produkt verbinden würde.

Das Bauteil beinhaltet mindestens eine Bauteiloberfläche. Mindestens ein Teil der Bauteiloberfläche dient bevorzugt dazu mit dem Klebstoff in Kontakt zu treten bzw. von dem Klebstoff überlagert zu werden. Über den an der Bauteiloberfläche befindlichen Klebstoff kann das Bauteil mit anderen Gegenständen oder der Haut eines Nutzers verbunden werden.

Der Klebstoff, der in dem Produkt enthalten ist, wird bevorzugt punktförmig, teilflächig oder vollflächig auf das Substrat oder mindestens einer Bauteiloberfläche eines Bauteils aufgebracht. Bevorzugt wird der Klebstoff beispielsweise in Form eines bestimmten Musters, bevorzugt eines wiederkehrenden oder replikativen Musters auf das Substrat oder das Bauteil, insbesondere auf mindestens einen Teil einer Bauteiloberfläche, aufgebracht. Bevorzugt wird der Klebstoff in Form einer Schicht auf das Substrat oder mindestens einer Bauteiloberfläche eines Bauteils aufgebracht. Bevorzugt ist der Klebstoff, der in dem Produkt enthalten ist in Form der ersten Schicht, wie sie im Zusammenhang mit der Klebstoffschicht beschrieben wurde, mit dem Produkt verbunden. Bevorzugt ist die Klebstoffschicht mit dem Substrat oder dem Bauteil direkt verbunden.

Vor, während oder nach der Verbindung des Substrats oder des Bauteils mit der Klebstoffschicht, kann die Klebstoffschicht auf einer ihrer Oberflächen mindestens eine erste weitere Schicht oder eine zweite weitere Schicht aufweisen. Weist die Klebstoffschicht vor der Verbindung mit dem Substrat oder dem Bauteil auf mindestens zwei Oberflächen jeweils eine weitere Schicht auf, so wird mindestens eine der weiteren Schichten vor Kontakt mit dem Substrat oder dem Bauteil entfernt. Die andere weitere Schicht, zum Beispiel die zweite weitere Schicht, kann bis zum Verbinden des Substrats oder des Bauteils mit einem Gegenstand oder der Haut eines Patienten zum Schutz der Klebstoffschicht auf dieser verbleiben. Die erst Schicht, die erste weitere Schicht sowie die zweite weitere Schicht sind bevorzugt jeweils auf die gleiche Weise aufgebaut und ausgestaltet, wie bereits oben im Zusammenhang mit der Klebstoffschicht beschrieben.

In einer bevorzugten Ausgestaltung des Produkts, ist das Produkt ausgewählt aus der Gruppe bestehend aus einem Pflaster, einem (Wund-)Verband, einem Tape, einem selbstklebenden Band, einem Stomabeutel für einen künstlichen Darmausgang, einer Blutaufnahmebinde, einer Bandage, einem medizinisches Gerät oder zumindest einem Bestandteil dieser Endprodukte.

Ein weiterer Gegenstand der Erfindung ist ein Kit aufweisend die Komponenten (V1) und (V2). Die Bestandteile, deren Verhältnisse sowie das Verfahren zu der Herstellung der Komponenten (V1) und (V2) entsprechen denen im Zusammenhang mit dem erfindungsgemäßen Klebstoff beschriebenen Komponenten A) bis L). Die Komponenten (V1) und (V2) werden bevorzugt entsprechend dem erfindungsgemäßen Verfahren zur Herstellung eines Schichtaufbaus, beinhaltend mindestens eine Klebstoffschicht verarbeitet.

### Beispiele:

Die Erfindung wird anhand der nachfolgenden Beispiele näher erläutert, ohne jedoch darauf beschränkt zu sein.

### Methoden:

Sofern nicht abweichend gekennzeichnet, beziehen sich alle Prozentangaben auf das Gewicht und die Gesamtmenge bzw. auf das Gesamtgewicht der Zusammensetzungen.

Sofern nicht abweichend vermerkt, beziehen sich alle analytischen Messungen auf Messungen bei Temperaturen von 23 °C.

Die Feststoff- bzw. Festkörpergehalte wurden entsprechend DIN EN ISO 3251 durch Erhitzen einer ausgewogenen Probe auf 105 °C bis zur Gewichtskonstanz ermittelt. Bei Gewichtskonstanz wurde durch erneutes Auswiegen der Probe der Festkörpergehalt berechnet.

NCO-Werte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23 °C mit einem Rotationsviskosimeter der Firma Anton Paar Germany GmbH, Ostfildern, DE bestimmt (1 Pa s = 1 N/m^{2∗}s).

Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

Der pH-Wert wurde gemäß der in DIN ISO 976 beschriebenen Methode an der unverdünnten Probe gemessen.

Die Glasübergangstemperatur T_{g} wurde mittels dynamischer Differenzkalorimetrie (DSC) in Anlehnung an die DIN EN 61006, Verfahren A, bestimmt, wobei ein DSC Gerät (Kalorimeter Pyris Diamond DSC von Perkin-Elmer) verwendet wird, das zur Bestimmung von T_{g} mit Indium und Blei kalibriert ist. Es werden 10 mg der zu untersuchenden Substanz in einen verschließbaren Aluminium-Tiegel eingewogen und dieser verschlossen. Es werden drei unmittelbar aufeinander folgende Durchläufe einer Aufheizungen von -100°C bis +150°C, Heizrate 20 K/min, mit anschließender Abkühlung Kühlrate 320 K/min vorgenommen und die dritte Aufheizkurve zur Bestimmung der Werte verwendet. Als T_{g} wird die Temperatur bei der halben Höhe einer Glasübergangsstufe bestimmt.

### Bestimmung der Schälkraft (90°-Abzugstest) nach DIN EN 1464

Die Bestimmung der Schälkraft erfolgte mit einer Zugprüfmaschine gemäß DIN EN ISO 527-1 und einer Rollenschälvorrichtung. Die zu untersuchende Klebstoffschicht wurde rückseitig mit einem Klebeband (TESA4104) verstärkt und passend auf 20 × 2cm² zugeschnitten. Die Trennpapierseite der Klebstoffschicht wird auf ein mit Aceton gereinigtes Alublech (Firma Krüppel aus Krefeld; reinst Aluminium 99,9%) (20 × 2 cm²) mit 3 Doppelhüben einer 4kg Walze geklebt. Nach 60 min Kontaktzeit zum Aluminiumsubstrat erfolgt die Bestimmung der Schälkraft nach DIN 1464 in einem Schälwinkel von 90° unter Trennung der Fügeteile. Die Schälgeschwindigkeit beträgt 300 mm/min. Die Schälkraft wird in N/20mm angegeben.

**Bestimmung der Wasserdampfdurchlässigkeit, auch MVTR (Moisture vapor transmission rate)**

Die Bestimmung der MVTR erfolgt in Anlehnung an die DIN EN13726-2 (Teil 3.2). Dabei wird ein Metallzylinder, wie in der DIN beschrieben mit Wasser gefüllt und durch den zu untersuchenden Film bzw. die zu untersuchende Schicht an der Oberseite verschlossen. Anschließend wird das Gesamtgewicht (Becher mit Wasser und Film) mittels Waage bestimmt. Der Messaufbau wird für 24 h bei 37°C gelagert und wiederum das Gewicht bestimmt. Durch Subtraktion lässt sich der Wasserverlust, der durch den Film verdampft, ermitteln. Die MVTR wird in g/(m^{2∗}24 h) angegeben.

### Verwendete Substanzen und Abkürzungen:

| | |
|---|---|
| Diaminosulfonat: | NH₂-CH₂CH₂-NH-CH₂CH₂-SO₃Na (45 %ig in Wasser) |
| PolyTHF 1000 | Poly(tetramethylenglykol)polyetherdiol mit der zahlenmittleren Molmasse von 1000 g/mol, BASF SE, Ludwigshafen, DE |
| PolyTHF 2000 | Poly(tetramethylenglykol)polyetherdiol mit der zahlenmittleren Molmasse von 2000 g/mol, BASF SE, Ludwigshafen, DE |
| PPG | Polypropylenglykol, Covestro AG, Leverkusen, DE. PPG wurde, soweit nicht anders angegeben, über KOH-Katalyse hergestellt. |
| Desmodur N 3300 | Aliphatisches Polyisocyanat (HDI-Isocyanurat), NCO-Gehalt ca. 21,8 %, Covestro AG, Leverkusen, Deutschland |
| Wasser | Durch Ionenaustauscher vollentsalztes Wasser |
| Baymedix^{®} FP520 | Hydrophiles, aliphatisches Polyisocyanat basierend auf Hexamethylendiisocyanat (HDI), Covestro AG, Leverkusen, Deutschland |

Die eingesetzten Isocyanate-Komponenten sind Handelsprodukte der Covestro Deutschland AG, Leverkusen, DE. Weitere Chemikalien von Sigma-Aldrich Chemie GmbH, Taufkirchen, DE. Die Rohstoffe wurden, soweit nicht anders erwähnt, ohne weitere Reinigung oder Vorbehandlung eingesetzt.

### Amorphe Polyurethanharnstoffdispersion 1 (V1)

60,0 g Polypropylenglykol mit einer zahlenmittleren Molmasse von 1000 g/mol und 280 g Polypropylenglykol mit einer zahlenmittleren Molmasse von 2000 g/mol wurden auf 65°C aufgeheizt. Anschließend wurde ein Gemisch aus 30,1 g Hexamethylendiisocyanat und 39,8 g Isophorondiisocyanat sowie 2 Tropfen Zinnoktanoat zugegeben und solange bei 130°C gerührt bis der theoretische NCO-Wert unterschritten war (ca. 90 min). Das fertige Präpolymer wurde mit 730 g Aceton bei 50°C gelöst und wurde anschließend bei 40°C eine Lösung aus 3,0 g Ethylendiamin, 18,9 g Diaminosulfonat, 3,6 g Diethanolamin und 74 g Wasser zudosiert. Die Nachrührzeit betrug 15 min. Danach wurde durch Zugabe von 550 g Wasser dispergiert. Es folgte die Entfernung des Lösemittels durch Destillation im Vakuum und es wurde eine lagerstabile Dispersion erhalten; der Festgehalt wurde durch Zusatz von Wasser eingestellt.

| | |
|---|---|
| Feststoffgehalt: | 41 % |
| Partikelgröße (LKS): | 160 nm |
| Viskosität: | 365 mPa s |

### Anwendungsbeispiel 1 (Vergleichsbeispiel):

100g der (erfindungsgemäßen) Polyurethanharnstoffdispersion 1 wurde mit 2g einer 10Gew.-% wässrigen Rheolate 208 Dispersion in einem Speedmixer-Becher vorgelegt. Die blasenfreie Vermischung zu einer Polyurethanharnstoffzusammensetzung erfolgt in dem Speedmixer bei einer Umdrehungszahl von 2750 min⁻¹ für 1 Minute. Nach der Applizierung mittels Ausstreichbalken der Firma Erichsen (200µm) auf ein Trennpapier der Firma Felix Schöller mit der Bezeichnung Y5900 erfolgte die Trocknung für 20 min bei 40°C und für 10 min bei 130°C.
MVTR: 1950 g/d m².
Schälkraftmessung: Kohäsionsbruch bereits bei kleiner Kraftaufwendung

### Anwendungsbeispiel 2 (erfindungsgemäß):

Filmherstellung der Polyurethanharnstoffdispersion 1 erfolgte wie Anwendungsbeispiel 1, jedoch nach dem Einbringen des Rheolates erfolgte die zusätzliche Einmischung von 1,9 Gew.-% Baymedix^{®} FP520 mittels Speedmixer bei 2750 min⁻¹ für 1min.
Schälkraftmessung: 8,8 N / 20mm

### Anwendungsbeispiel 3 (erfindungsgemäß):

Filmherstellung der Polyurethanharnstoffdispersion 1 erfolgte wie Anwendungsbeispiel 2, jedoch bei einer Zugabe von 3,8 Gew.-% Baymedix^{®} FP520.
Schälkraftmessung: 5,8 N / 20mm

### Anwendungsbeispiel 4 (erfindungsgemäß):

Filmherstellung der Polyurethanharnstoffdispersion 1 erfolgte wie Anwendungsbeispiel 2, jedoch bei einer Zugabe von 6,8 Gew.-% Baymedix^{®} FP520.
Schälkraftmessung: 1,5 N / 20mm

Wie anhand der Beispiele zu erkennen ist, kann die Schälkraft der aus dem erfindungsgemäßen Klebstoff hergestellten Klebstoffschichten gezielt durch Wahl der Menge an beigemischter Komponente (V2) in Form von Baymedix^{®} FP520 als Polyisocyanat eingestellt werden. Höhere Mengen (V2) führen zu niedrigerer Schälkraft. So ist es möglich, die Klebkraft der Komponente (V1), die für sensitive Anwendungen wie Verklebung auf Haut oder Wunden zu hoch ist, maßgeschneidert für jede Anwendung einzustellen, ohne dass wie im Stand der Technik ein kompliziertes Verarbeitungsverfahren mit zusätzlichen Schritten nötig ist. Die im Stand der Technik verwendeten Klebstoffsysteme, die eine einstellbare Klebkraft ermöglichen, sind Zweikomponenten-Reaktivsysteme. Diese haben einmal gemischt nur eine sehr begrenzte Verarbeitungszeit, da direkt nach Mischen die Polymerisation bzw. Vernetzung einsetzt und sich damit kontinuierlich das Fließ- und Beschichtungsverhalten der Mischung ändert. Insofern können diese nur durch eine Inline-Mischtechnik und unter erhöhtem Reinigungsaufwand des Auftragswerks, verbunden mit Prozessunterbrechungen, hergestellt werden.

## Patentansprüche

1. Ein Klebstoff, herstellbar aus einer wässrigen Polyurethanharnstoff-Dispersion, enthaltend
(V1) einen Polyurethanharnstoff, welcher erhältlich ist, durch Umsetzung von mindestens
A) einer aliphatischen, cycloaliphatischen oder araliphatischen Polyisocyanat-Komponente mit einer mittleren Isocyanatfunktionalität von ≥ 1,8 und ≤ 2,6,
B) einer polymeren Polyether-Polyol-Komponente,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls weitere polymere Polyole, welche unterschiedlich sind von B),
G) gegebenenfalls einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert, und
H) gegebenenfalls einer aliphatischen, cycloaliphatischen oder araliphatischen Polyisocyanat-Komponente mit einer mittleren Isocyanatfunktionalität von > 2,6 und ≤ 4,
wobei die Komponenten B) und F) zusammen ≤ 30 Gew.-% an Komponente F), bezogen auf die Gesamtmasse der Komponenten B) und F) enthalten, sowie
(V2) ein hydrophiles Polyisocyanat, herstellbar mindestens aus den Komponenten
I) eine aliphatische, cycloaliphatische oder araliphatische Polyisocyanat-Komponente mit einer mittleren Isocyanatfunktionalität von bevorzugt ≥ 2,0 und ≤ 3,6,
J) eine polymere, hydrophile und monofunktionelle Polyalkylenoxid -Komponente,
K) gegebenenfalls weitere hydrophilierenden Komponenten, die unterschiedlich sind von J),
L) gegebenenfalls Zuschlags- und Hilfsstoffen.

2. Der Klebstoff nach Anspruch 1, wobei die Komponente A) Isophorondiisocyanat, Hexamethylendiisocyanat oder ein Gemisch aus Isophorondiisocyanat und Hexamethylendiisocyanat ist.

3. Der Klebstoff nach einem der Ansprüche 1 oder 2, wobei die Komponente B) Poly(propylenglykol)polyetherpolyole enthält oder daraus besteht.

4. Der Klebstoff nach einem der Ansprüche 1 bis 3, wobei die Komponente B) eine mittlere Molekularmasse in einem Bereich von 400 bis 4000 g/mol aufweist.

5. Der Klebstoff nach einem der Ansprüche 1 bis 4, wobei die Komponente B) ein Gemisch aus Poly(propylenglykol)polyetherpolyolen enthält oder daraus besteht, wobei sich die Poly(propylenglykol)polyetherpolyole in ihren zahlenmittleren Molekulargewichten um wenigstens 100 g/mol unterscheiden.

6. Der Klebstoff nach einem der Ansprüche 1 bis 5, wobei die Komponente C) wenigstens eine aminofunktionelle Verbindung C1), die keine ionischen oder ionogenen Gruppen aufweist und eine aminofunktionelle Verbindung C2), die ionische oder ionogene Gruppen aufweist enthält.

7. Der Klebstoff nach einem der Ansprüche 1 bis 6, wobei es sich bei der Komponente D) um nichtionisch hydrophilierende Komponenten D1) handelt.

8. Der Klebstoff nach einem der Ansprüche 1 bis 7, wobei der Polyurethanharnstoff erhältlich ist indem isocyanatfunktionelle Polyurethanpräpolymere a) aus den Komponenten A), B) und gegebenenfalls D) und/oder C2), sowie gegebenenfalls den Verbindungen E) und/oder H) hergestellt werden und deren freie NCO-Gruppen anschließend ganz oder teilweise mit der aminofunktionellen Kettenverlängerer-Komponente C), sowie der Komponente G) und gegebenenfalls der Komponente D) umgesetzt werden.

9. Der Klebstoff nach einem der Ansprüche 1 bis 8, wobei der Polyurethanharnstoff einen Tg ≤ - 25 °C aufweist.

10. Der Klebstoff nach einem der vorhergehenden Ansprüche, wobei
I) ausgewählt ist aus der Gruppe bestehend aus
I1.) niedermolekularen, aliphatischen, cycloaliphatischen oder araliphatischen Diisocyanaten einer Molmasse von 140 bis 278 g/mol;
I2.) aus I1.) herstellbare Polyisocyanate mit einer Isocyanatfunktionalität von 2 bis 3,6;
I3.) einer Kombination aus I1.) und I2.);
J) ausgewählt ist aus der Gruppe bestehend aus
J1.) einem monofunktionellen Polyalkylenoxid einer OH-Zahl von 10 bis 250;
J2.) Ethylenoxid, Propylenoxid, Butylenoxid, Pentylenoxid oder einer Mischung aus mindestens zwei hiervon;
J3.) einem monofunktionellen Polyalkylenoxid mit einem Ethylenoxidanteil von 50 bis 100 mol% bezogen auf die Gesamtmenge der enthaltenen Oxyalkylengruppen ist
J4.) einer Kombination aus mindestens zwei von J1.) bis J3.).

11. Eine wässrige Polyurethanharnstoff-Dispersion enthaltend
(V1) einen amorphen Polyurethanharnstoff, welcher erhältlich ist durch Umsetzung von mindestens
A) einer aliphatischen, cycloaliphatischen oder araliphatischen Polyisocyanat-Komponente mit einer mittleren Isocyanatfunktionalität von ≥ 1,8 und ≤ 2,6,
B) einer polymeren Polyether-Polyol-Komponente,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls weitere polymere Polyolen, welche unterschiedlich sind von B)
G) gegebenenfalls einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
H) gegebenenfalls einer aliphatischen, cycloaliphatischen oder araliphatischen Polyisocyanat-Komponente mit einer mittleren Isocyanatfunktionalität von > 2,6 und ≤ 4,
wobei die Komponenten B) und F) zusammen ≤ 30 Gew.-% an Komponente F), bezogen auf die Gesamtmasse der Komponenten B) und F) enthalten
sowie
(V2) ein hydrophiles Polyisocyanat, herstellbar aus
I) einer aliphatischen, cycloaliphatischen oder araliphatischen Polyisocyanat-Komponente mit einer mittleren Isocyanatfunktionalität von bevorzugt ≥ 2,0 und ≤ 3,6,
J) einer polymeren, hydrophilen und monofunktionellen Polyalkylenoxid -Komponente,
K) gegebenenfalls weiteren hydrophilierenden Komponenten, die unterschiedlich sind von J),
L) gegebenenfalls Zuschlags- und Hilfsstoffen.

12. Ein Verfahren zur Herstellung eines Schichtaufbaus beinhaltend mindestens eine Klebstoffschicht, umfassend mindestens die Schritte:
(I) Mischen eines hydrophilen Polyisocyanats (V2) zu einem Polyurethanharnstoff (V1) unter Erhalt einer Polyurethanharnstoff-Dispersion gemäß Anspruch 11,
(II) Aufbringen der Polyurethanharnstoff-Dispersion aus Schritt (I) auf eine erste weitere Schicht, unter Erhalt eines Vorläufers,
(III) thermische Behandlung des Vorläufers aus Schritt (II) bei Temperaturen in einem Bereich von 20 °C bis 200 °C, unter Bildung der Klebstoffschicht.

13. Das Verfahren nach Anspruch 12, umfassend mindestens einen der folgenden weiteren Schritte:
(IV) Ablösen der Klebstoffschicht von der ersten weiteren Schicht;
(V) Übertragen der Klebstoffschicht von der ersten weiteren Schicht auf eine zweite weitere Schicht;
(VI) Überdecken der Klebstoffschicht mit einer zweiten weiteren Schicht auf der ersten Oberfläche der ersten Schicht;
(VII) Überdecken der Klebstoffschicht mit einer zweiten weiteren Schicht auf der ersten weiteren Oberfläche der ersten Schicht;
(VIII) Übertragen der Klebstoffschicht von der ersten weiteren Schicht auf ein Substrat;
(IX) Übertragen der Klebstoffschicht von der ersten weiteren Schicht auf mindestens einen Teil einer Bauteiloberfläche eines Bauteils;
(X) Übertragen der Klebstoffschicht von der ersten weiteren Schicht auf eine dritte weitere Schicht.

14. Verwendung eines Klebstoffs nach einem der Ansprüche 1 bis 10 oder einer Klebstoffschicht nach einem der Ansprüche 11 bis 14 zur Befestigung eines Produktes auf einem Gegenstand oder der Haut eines Lebewesens.

15. Ein Kit, aufweisend mindestens die Komponenten:
(V1) einen amorphen Polyurethanharnstoff, welcher erhältlich ist durch Umsetzung von mindestens
A) einer aliphatischen, cycloaliphatischen oder araliphatischen Polyisocyanat-Komponente mit einer mittleren Isocyanatfunktionalität von ≥ 1,8 und ≤ 2,6,
B) einer polymeren Polyether-Polyol-Komponente,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls weitere polymere Polyolen, welche unterschiedlich sind von B),
G) gegebenenfalls einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert, und
H) gegebenenfalls einer aliphatischen, cycloaliphatischen oder araliphatischen Polyisocyanat-Komponente mit einer mittleren Isocyanatfunktionalität von > 2,6 und ≤ 4,
wobei die Komponenten B) und F) zusammen ≤ 30 Gew.-% an Komponente F), bezogen auf die Gesamtmasse der Komponenten B) und F) enthalten
sowie
(V2) ein hydrophiles Polyisocyanat herstellbar aus
I) einer aliphatischen, cycloaliphatischen oder araliphatischen Polyisocyanat-Komponente mit einer mittleren Isocyanatfunktionalität von bevorzugt ≥ 2,0 und ≤ 3,6,
J) einer polymeren, hydrophilen und monofunktionellen Polyalkylenoxid -Komponente,
K) gegebenenfalls weiteren hydrophilierenden Komponenten, die unterschiedlich sind von J),
L) gegebenenfalls Zuschlags- und Hilfsstoffen.

## Claims

1. Adhesive producible from an aqueous polyurethaneurea dispersion comprising
(V1) a polyurethaneurea obtainable by reacting at least
A) one aliphatic, cycloaliphatic or araliphatic polyisocyanate component having an average isocyanate functionality of ≥ 1.8 and ≤ 2.6,
B) one polymeric polyetherpolyol component,
C) one amino-functional chain extender component having at least 2 isocyanate-reactive amino groups, containing at least one amino-functional compound C1) that does not have any ionic or ionogenic groups and/or an amino-functional compound C2) that has ionic or ionogenic groups,
D) optionally further hydrophilizing components different than C2),
E) optionally hydroxy-functional compounds having a molecular weight of 62 to 399 mol/g,
F) optionally further polymeric polyols that are different than B),
G) optionally one compound having exactly one isocyanate-reactive group or one compound having more than one isocyanate-reactive group, where only one of the isocyanate-reactive groups reacts with the isocyanate groups present in the reaction mixture under the reaction conditions chosen, and
H) optionally one aliphatic, cycloaliphatic or araliphatic polyisocyanate component having an average isocyanate functionality of > 2.6 and ≤ 4,
where components B) and F) together contain ≤ 30% by weight of component F), based on the total mass of components B) and F), and
(V2) a hydrophilic polyisocyanate preparable at least from the components of
I) an aliphatic, cycloaliphatic or araliphatic polyisocyanate component having an average isocyanate functionality of preferably ≥ 2.0 and ≤ 3.6,
J) a polymeric, hydrophilic and monofunctional polyalkylene oxide component,
K) optionally further hydrophilizing components different than J),
L) optionally admixtures and auxiliaries.

2. Adhesive according to Claim 1, wherein component A) is isophorone diisocyanate, hexamethylene diisocyanate or a mixture of isophorone diisocyanate and hexamethylene diisocyanate.

3. Adhesive according to either of Claims 1 and 2, wherein component B) contains or consists of poly(propylene glycol) polyetherpolyols.

4. Adhesive according to any of Claims 1 to 3, wherein component B) has an average molar mass within a range from 400 to 4000 g/mol.

5. Adhesive according to any of Claims 1 to 4, wherein component B) contains or consists of a mixture of poly(propylene glycol) polyetherpolyols, where the poly(propylene glycol) polyetherpolyols differ by at least 100 g/mol in their number-average molecular weights.

6. Adhesive according to any of Claims 1 to 5, wherein component C) comprises at least one amino-functional compound C1) having no ionic or ionogenic groups and one amino-functional compound C2) having ionic or ionogenic groups.

7. Adhesive according to any of Claims 1 to 6, wherein component D) comprises nonionically hydrophilizing components D1).

8. Adhesive according to any of Claims 1 to 7, wherein the polyurethaneurea is obtainable by preparing isocyanate-functional polyurethane prepolymers a) from components A), B) and optionally D) and/or C2), and optionally compounds E) and/or H), and the free NCO groups thereof are then wholly or partially reacted with the amino-functional chain-extender component C), and also component G) and optionally component D).

9. Adhesive according to any of Claims 1 to 8, wherein the polyurethaneurea has a Tg ≤ -25°C.

10. Adhesive according to any of the preceding claims, wherein
I) is selected from the group consisting of
I1.) low molecular weight aliphatic, cycloaliphatic **or araliphatic** diisocyanates of molar mass from 140 to 278 g/mol;
I2.) polyisocyanates preparable from I1.) and having an isocyanate functionality of 2 to 3.6;
I3.) a combination of I1.) and I2.);
J) is selected from the group consisting of
J1.) a monofunctional polyalkylene oxide of OH number from 10 to 250;
J2.) ethylene oxide, propylene oxide, butylene oxide, pentylene oxide or a mixture of at least two of these;
J3.) a monofunctional polyalkylene oxide having an ethylene oxide content of 50 to 100 mol% based on the total amount of oxyalkylene groups present
J4.) a combination of at least two of J1.) to J3.).

11. Aqueous polyurethaneurea dispersion comprising
(V1) an amorphous polyurethaneurea obtainable by reacting at least
A) one aliphatic, cycloaliphatic or araliphatic polyisocyanate component having an average isocyanate functionality of ≥ 1.8 and ≤ 2.6,
B) one polymeric polyetherpolyol component,
C) one amino-functional chain extender component having at least 2 isocyanate-reactive amino groups, containing at least one amino-functional compound C1) that does not have any ionic or ionogenic groups and/or an amino-functional compound C2) that has ionic or ionogenic groups,
D) optionally further hydrophilizing components different than C2),
E) optionally hydroxy-functional compounds having a molecular weight of 62 to 399 mol/g,
F) optionally further polymeric polyols that are different than B),
G) optionally one compound having exactly one isocyanate-reactive group or one compound having more than one isocyanate-reactive group, where only one of the isocyanate-reactive groups reacts with the isocyanate groups present in the reaction mixture under the reaction conditions chosen, and
H) optionally one aliphatic, cycloaliphatic or araliphatic polyisocyanate component having an average isocyanate functionality of ≥ 2.6 and ≤ 4,
where components B) and F) together contain ≤ 30% by weight of component F), based on the total mass of components B) and F),
and
(V2) a hydrophilic polyisocyanate preparable from
I) an aliphatic, cycloaliphatic or araliphatic polyisocyanate component having an average isocyanate functionality of preferably ≥ 2.0 and ≤ 3.6,
J) a polymeric, hydrophilic and monofunctional polyalkylene oxide component,
K) optionally further hydrophilizing components different than J),
L) optionally admixtures and auxiliaries.

12. Process for producing a layer construction including at least one adhesive layer, comprising at least the steps of:
(I) mixing a hydrophilic polyisocyanate (V2) into a polyurethaneurea (V1) to obtain a polyurethaneurea dispersion according to Claim 11,
(II) applying the polyurethaneurea dispersion from step (I) to a first further layer to obtain a precursor,
(III) thermally treating the precursor from step (II) at temperatures within a range from 20°C to 200°C to form the adhesive layer.

13. Process according to Claim 12, comprising at least one of the following further steps:
(IV) detaching the adhesive layer from the first further layer;
(V) transferring the adhesive layer from the first further layer to a second further layer;
(VI) covering the adhesive layer with a second further layer on the first surface of the first layer;
(VII) covering the adhesive layer with a second further layer on the first further surface of the first layer;
(VIII) transferring the adhesive layer from the first further layer to a substrate;
(IX) transferring the adhesive layer from the first further layer to at least a portion of a component surface of a component;
(X) transferring the adhesive layer from the first further layer to a third further layer.

14. Use of an adhesive according to any of Claims 1 to 10 or of an adhesive layer according to any of Claims 11 to 14 for securing a product on an article or on the skin of a living being.

15. Kit, having at least the components of:
(V1) an amorphous polyurethaneurea obtainable by reacting at least
A) one aliphatic, cycloaliphatic or araliphatic polyisocyanate component having an average isocyanate functionality of ≥ 1.8 and ≤ 2.6,
B) one polymeric polyetherpolyol component,
C) one amino-functional chain extender component having at least 2 isocyanate-reactive amino groups, containing at least one amino-functional compound C1) that does not have any ionic or ionogenic groups and/or an amino-functional compound C2) that has ionic or ionogenic groups,
D) optionally further hydrophilizing components different than C2),
E) optionally hydroxy-functional compounds having a molecular weight of 62 to 399 mol/g,
F) optionally further polymeric polyols that are different than B),
G) optionally one compound having exactly one isocyanate-reactive group or one compound having more than one isocyanate-reactive group, where only one of the isocyanate-reactive groups reacts with the isocyanate groups present in the reaction mixture under the reaction conditions chosen, and
H) optionally one aliphatic, cycloaliphatic or araliphatic polyisocyanate component having an average isocyanate functionality of > 2.6 and ≤ 4,
where components B) and F) together contain ≤ 30% by weight of component F), based on the total mass of components B) and F)
and
(V2) a hydrophilic polyisocyanate preparable from
I) an aliphatic, cycloaliphatic or araliphatic polyisocyanate component having an average isocyanate functionality of preferably ≥ 2.0 and ≤ 3.6,
J) a polymeric, hydrophilic and monofunctional polyalkylene oxide component,
K) optionally further hydrophilizing components different than J),
L) optionally admixtures and auxiliaries.

## Revendications

1. Adhésif pouvant être fabriqué à partir d'une dispersion aqueuse de polyuréthane-urée, contenant
(V1) une polyuréthane-urée, qui peut être obtenue par réaction d'au moins
A) un composant polyisocyanate aliphatique, cycloaliphatique ou araliphatique ayant une fonctionnalité isocyanate moyenne ≥ 1,8 et ≤ 2,6,
B) un composant polyéther-polyol polymère,
C) un composant extenseur de chaîne amino-fonctionnel ayant au moins 2 groupes amino réactifs vis-à-vis des isocyanates, contenant au moins un composé amino-fonctionnel C1), qui ne comprend pas de groupes ioniques ou ionogènes et/ou un composé amino-fonctionnel C2), qui comprend des groupes ioniques ou ionogènes,
D) éventuellement des composants hydrophilisants supplémentaires, qui sont différents de C2),
E) éventuellement des composés hydroxy-fonctionnels ayant une masse moléculaire de 62 à 399 mole/g,
F) éventuellement des polyols polymères supplémentaires qui sont différents de B),
G) éventuellement un composé qui comprend exactement un groupe réactif vis-à-vis des isocyanates, ou un composé qui comprend plus d'un groupe réactif vis-à-vis des isocyanates, un seul des groupes réactifs vis-à-vis des isocyanates réagissant, dans les conditions de réaction choisies, avec les groupes isocyanates présents dans le mélange réactionnel,
et
H) éventuellement un composant polyisocyanate aliphatique, cycloaliphatique ou araliphatique ayant une fonctionnalité isocyanate moyenne > 2,6 et ≤ 4,
les composants B) et F) contenant conjointement ≤ 30 % en poids du composant F) par rapport à la masse totale des composants B) et F), ainsi que
(V2) un polyisocyanate hydrophile, pouvant être fabriqué au moins à partir des composants
I) un composant polyisocyanate aliphatique, cycloaliphatique ou araliphatique ayant une fonctionnalité isocyanate moyenne, de préférence ≥ 2,0 et ≤ 3,6,
J) un composant poly(oxyde d'alkylène) polymère, hydrophile et monofonctionnel,
K) éventuellement des composants hydrophilisants supplémentaires qui sont différents de J),
L) éventuellement des additifs et des adjuvants.

2. Adhésif selon la revendication 1, dans lequel le composant A) est le diisocyanate d'isophorone, le diisocyanate d'hexaméthylène ou un mélange de diisocyanate d'isophorone et de diisocyanate d'hexaméthylène.

3. Adhésif selon l'une des revendications 1 ou 2, dans lequel le composant B) contient des poly(propylèneglycol)polyétherpolyols ou en est constitué.

4. Adhésif selon l'une des revendications 1 à 3, dans lequel le composant B) présente une masse moléculaire moyenne dans la plage de 400 à 4 000 g/mole.

5. Adhésif selon l'une des revendications 1 à 4, dans lequel le composant B) contient un mélange de poly(propylèneglycol)polyétherpolyols ou en est constitué, les poly(propylèneglycol)polyétherpolyols ayant des masses moléculaires moyennes en nombre qui diffèrent d'au moins 100 g/mole.

6. Adhésif selon l'une des revendications 1 à 5, dans lequel le composant C) contient au moins un composé amino-fonctionnel C1) qui ne comprend pas de groupes ioniques ou ionogènes et un composé amino-fonctionnel C2) qui comprend des groupes ioniques ou ionogènes.

7. Adhésif selon l'une des revendications 1 à 6, dans lequel, pour ce qui concerne le composant D), il s'agit de composants hydrophilisants non ioniques D1).

8. Adhésif selon l'une des revendications 1 à 7, dans lequel la polyuréthane-urée peut être obtenue par fabrication de prépolymères de polyuréthane à fonctionnalité isocyanate a) à partir des composants A), B) et éventuellement D) et/ou C2), éventuellement à partir des composés E) et/ou H), et dont les groupes NCO libres sont ensuite mis à réagir en totalité ou en partie avec le composant extenseur de chaîne amino-fonctionnel C), ainsi qu'avec le composant G) et éventuellement le composant D).

9. Adhésif selon l'une des revendications 1 à 8, dans lequel la polyuréthane-urée présente une Tg ≤ -25 °C.

10. Adhésif selon l'une des revendications précédentes, dans lequel
I) est choisi dans le groupe consistant en
I1.) lesdits isocyanates aliphatiques, cycloaliphatiques ou araliphatiques à faible masse moléculaire ayant une masse moléculaire de 140 à 278 g/mole ;
I2.) les polyisocyanates pouvant être fabriqués à partir de I1.), ayant une fonctionnalité isocyanate de 2 à 3,6 ;
I3.) une combinaison de I1.) et de I2.) ;
J) est choisi dans le groupe consistant en
J1.) un poly(oxyde d'alkylène) monofonctionnel ayant un indice d'OH de 10 à 250 ;
J2.) l'oxyde d'éthylène, l'oxyde de propylène, l'oxyde de butylène, l'oxyde de pentylène ou un mélange d'au moins deux d'entre eux ;
J3.) un poly(oxyde d'alkylène) monofonctionnel ayant une proportion d'oxyde d'éthylène de 50 à 100 % en moles par rapport à la totalité des groupes oxyalkylène présents ;
J4.) une combinaison d'au moins deux de J1.) à J3.).

11. Dispersion aqueuse de polyuréthane-urée contenant
(V1) une polyuréthane-urée, qui peut être obtenue par réaction d'au moins
A) un composant polyisocyanate aliphatique, cycloaliphatique ou araliphatique ayant une fonctionnalité isocyanate moyenne ≥ 1,8 et ≤ 2,6,
B) un composant polyéther-polyol polymère,
C) un composant extenseur de chaîne amino-fonctionnel ayant au moins 2 groupes amino réactifs vis-à-vis des isocyanates, contenant au moins un composé amino-fonctionnel C1), qui ne comprend pas de groupes ioniques ou ionogènes et/ou un composé amino-fonctionnel C2), qui comprend des groupes ioniques ou ionogènes,
D) éventuellement des composants hydrophilisants supplémentaires, qui sont différents de C2),
E) éventuellement des composés hydroxy-fonctionnels ayant une masse moléculaire de 62 à 399 mole/g,
F) éventuellement des polyols polymères supplémentaires qui sont différents de B),
G) éventuellement un composé qui comprend exactement un groupe réactif vis-à-vis des isocyanates, ou un composé qui comprend plus d'un groupe réactif vis-à-vis des isocyanates, un seul des groupes réactifs vis-à-vis des isocyanates réagissant, dans les conditions de réaction choisies, avec les groupes isocyanates présents dans le mélange réactionnel, et
H) éventuellement un composant polyisocyanate aliphatique, cycloaliphatique ou araliphatique ayant une fonctionnalité isocyanate moyenne > 2,6 et ≤ 4,
les composants B) et F) contenant conjointement ≤ 30 % en poids du composant F) par rapport à la masse totale des composants B) et F), ainsi que
(V2) un polyisocyanate hydrophile, pouvant être fabriqué au moins à partir des composants
I) un composant polyisocyanate aliphatique, cycloaliphatique ou araliphatique ayant une fonctionnalité isocyanate moyenne, de préférence ≥ 2,0 et ≤ 3,6,
J) un composant poly(oxyde d'alkylène) polymère, hydrophile et monofonctionnel,
K) éventuellement des composants hydrophilisants supplémentaires qui sont différents de J),
L) éventuellement des additifs et des adjuvants.

12. Procédé de fabrication d'un système de couches contenant au moins une couche adhésive, comprenant au moins les étapes :
(I) mélange d'un polyisocyanate hydrophile (V2) avec une polyuréthane-urée (V1) avec obtention d'une dispersion de polyuréthane-urée selon la revendication 11,
(II) application de la dispersion de polyuréthane-urée de l'étape (I) sur une première couche supplémentaire avec obtention d'un précurseur,
(III) traitement thermique du précurseur de l'étape (II) à des températures dans la plage de 20 °C à 200 °C, avec formation de la couche adhésive.

13. Procédé selon la revendication 12, comprenant au moins l'une des étapes supplémentaires suivantes :
(IV) détachement de la couche adhésive de la première couche supplémentaire ;
(V) transfert de la couche adhésive de la première couche supplémentaire sur une deuxième couche supplémentaire ;
(VI) recouvrement de la couche adhésive avec une deuxième couche supplémentaire sur la première surface de la première couche ;
(VII) recouvrement de la couche adhésive avec une deuxième couche supplémentaire sur la première surface supplémentaire de la première couche ;
(VIII) transfert de la couche adhésive de la première couche supplémentaire sur un substrat ;
(IX) transfert de la couche adhésive de la première couche supplémentaire sur au moins une partie d'une surface d'un composant ;
(X) transfert de la couche adhésive de la première couche supplémentaire sur la troisième couche supplémentaire.

14. Utilisation d'un adhésif selon l'une des revendications 1 à 10 ou d'une couche adhésive selon l'une des revendications 11 à 14 pour fixer un produit sur un objet ou sur la peau d'un être vivant.

15. Kit comportant au moins les composants :
(V1) une polyuréthane-urée, qui peut être obtenue par réaction d'au moins
A) un composant polyisocyanate aliphatique, cycloaliphatique ou araliphatique ayant une fonctionnalité isocyanate moyenne ≥ 1,8 et ≤ 2,6,
B) un composant polyéther-polyol polymère,
C) un composant extenseur de chaîne amino-fonctionnel ayant au moins 2 groupes amino réactifs vis-à-vis des isocyanates, contenant au moins un composé amino-fonctionnel C1), qui ne comprend pas de groupes ioniques ou ionogènes et/ou un composé amino-fonctionnel C2), qui comprend des groupes ioniques ou ionogènes,
D) éventuellement des composants hydrophilisants supplémentaires, qui sont différents de C2),
E) éventuellement des composés hydroxy-fonctionnels ayant une masse moléculaire de 62 à 399 mole/g,
F) éventuellement des polyols polymères supplémentaires qui sont différents de B),
G) éventuellement un composé qui comprend exactement un groupe réactif vis-à-vis des isocyanates, ou un composé qui comprend plus d'un groupe réactif vis-à-vis des isocyanates, un seul des groupes réactifs vis-à-vis des isocyanates réagissant, dans les conditions de réaction choisies, avec les groupes isocyanates présents dans le mélange réactionnel, et
H) éventuellement un composant polyisocyanate aliphatique, cycloaliphatique ou araliphatique ayant une fonctionnalité isocyanate moyenne > 2,6 et ≤ 4,
les composants B) et F) contenant conjointement ≤ 30 % en poids du composant F) par rapport à la masse totale des composants B) et F), ainsi que
(V2) un polyisocyanate hydrophile, pouvant être fabriqué au moins à partir des composants
I) un composant polyisocyanate aliphatique, cycloaliphatique ou araliphatique ayant une fonctionnalité isocyanate moyenne, de préférence ≥ 2,0 et ≤ 3,6,
J) un composant poly(oxyde d'alkylène) polymère, hydrophile et monofonctionnel,
K) éventuellement des composants hydrophilisants supplémentaires qui sont différents de J),
L) éventuellement des additifs et des adjuvants.
